# EUROPEAN PATENT APPLICATION

(11) **EP 2 840 139 A1**
(43) Date of publication of application: **25.02.2015**
(21) Application number: 13181473.3
(22) Date of filing: 23.08.2013
(51) Int. Cl.: C12N 15/10, C12N 15/66

(54) **Method for the single step introduction of a plurality of genes in microbial cells**

(71) Applicant: Wageningen Universiteit, 6708 PB Wageningen (NL)
(72) Inventor: van der Straat, Laura, 6708SJ Wageningen (NL); de Graaff, Leendert Hendrik, 6881 RS Velp (NL)
(74) Representative: Nederlandsch Octrooibureau

(57) **Abstract**

The invention provides a method for producing a vector for introducing a plurality of genes into a host cell, the method comprising the steps of: a) providing for each gene of the plurality of genes, a DNA fragment comprising an expression cassette for expression of the gene in the host cell, wherein the DNA fragment has staggered 5' and 3' ends that are part of a restriction enzyme recognition sequence, which recognition sequence is not present elsewhere in the DNA fragment and which recognition sequence at the 5' end of the DNA fragment differs from the recognition sequence at the 3' end of the DNA fragment, and wherein the overhang of the restriction enzyme recognition sequence at the 5' end is complementary with the overhang of the restriction enzyme recognition sequence at the 3' end; and b) combining the DNA fragments as obtained in a) by insertion of the DNA fragments into a unique restriction enzyme recognition site in a suitable vector, wherein the unique restriction enzyme recognition site in the vector has the same sequence as one of the two restriction recognition sequences at the 5' and 3' ends of the fragments in a), and wherein combination of a DNA fragment with a further DNA fragment produces a dysfunctional restriction enzyme site at the fusion site of the fragments. The DNA fragment comprising the expression cassette is preferably assembled from subfragments comprising functional elements that together form the expression cassette, which subfragments have staggered 5' and 3' ends, the overhang at the 5' end of each subfragment differs from the overhang at its 3' end, and the overhang at the 3' end of the subfragments comprising one type of element is complementary with the overhang at the 5' end of the subfragments comprising another type of element to allow assembly and operable linkage of the subfragments in a 5' to 3'order to form the functional expression cassette. The invention further provides a method for introducing a plurality of genes into a host cell, preferably into a filamentous fungal host cell, the method comprising the step of transforming the host cell with a vector obtainable by the above methods, in a single step.

## Description

### Field of the invention

The invention relates to the field of genetic modification of microorganisms, preferably filamentous fungi, in particular by the one step introduction of a plurality of genes, for instance constituting a complete metabolic pathway, into microbial cells. The invention further relates to use of the genetically modified microbial cells for production of a compound of interest.

### Background of the invention

Filamentous fungi are used for the industrial production of bio-based compounds like organic acids and for the production of enzymes that are important tools for bio-based processes. The exploitation of filamentous fungi results in more sustainable production processes. A major advantage of filamentous fungi over yeasts and bacteria is that they are able to convert all kind of different polysaccharide substrates and sugars, for instance the sugars from plant waste material, into valuable products. For the production of many products like organic acids and enzymes, filamentous fungi are more preferred as a host organism than bacteria or yeast.

Within the field of synthetic biology there is a growing interest in the use of filamentous fungi for the biosynthesis of new products that need the introduction of a plurality of genes or even complete biosynthetic pathways. This need for pathway transfer is recognized in literature, see e.g. the recent publication of Medema et al. (2011). However, such use of filamentous fungi as production hosts has major limitations, since the presently used methods do not accommodate the introduction of a plurality of genes (typically five or more) or of large biosynthetic pathways in a single step into the fungal genome. When done in a step-by-step manner, a disadvantage is that every next step requires a new transformation selection marker or the recycling of the marker used in the earlier step, implicating that this manner is limited by the number of available selection markers for filamentous fungi. This implies that only four to six genes can be introduced into the fungal host in such a step-by-step manner. In addition, the process is extremely time consuming. The introduction of five genes in this manner may require up to six months. This limits the ability to transfer complete pathways from a donor organism to the fungus of choice.

There thus is a need for a vector system that allows for the introduction of a large number of genes and/or of large biosynthetic pathways into a fungal genome in a single step.

Use of cosmid vectors in transformation of filamentous fungi has been described previously. However, their use is restricted to construct libraries for e.g. complementation of mutations (Timberlake et al., 1985) or for genome walking experiments (Orbach, 1994). Also there is an example of a cosmid that was isolated from *Monascus purpureus* and used to express a pathway in an *Aspergillus* species, however, without manipulating the gene structure of the pathway (Sakai, Kinoshita, Shimizu, & Nihira, 2008).

WO2012/051327 discloses methods of assembling a plurality of genetic units to form synthetic genetic constructs. The methods involve appending universal adapter oligonucleotides and flexible adapter oligonucleotides to the 5' and 3' ends of separate genetic units to be assembled to form separate dual extended genetic units. The dual extended genetic units are assembled together via homologous recombination between the flexible adapter oligonucleotide portions of the dual extended units to form synthetic genetic constructs.

US2012/258487 provides methods and compositions for assembling biological constructs (e.g., plasmids, transformed cells, etc.). In certain embodiments the methods involve encapsulating separate components of said biological construct each in a fluid droplet confined in a fluid channel; optionally mixing droplets from different fluid channels for a sequenced order of droplets carrying different components of said biological construct in a channel or chamber; and optionally combining two or more droplets each containing different components of said biological construct to permit said components to react with each other in one or more reactions contributing to the assembly of said biological construct. The BioBrick method as referred to in US2012/258487 is described to be applicable to the assembly of any DNA fragment that is nominally associated with a biological function. When applied to functional elements like a promoter or terminator, there is no possibility to exchange such functional elements (bricks) in the vector, when an element is inserted in the vector it is fixed.

There is however still a need in the art for means and methods allowing the introduction of a (large) plurality of genes, e.g. genes of a complicated biosynthetic pathway, into the genome of a host cell in a single step. It is an object of the present invention to provide for such means and methods.

### Summary of the invention

In a first aspect, the invention relates to a method for producing a vector for introducing a plurality of genes into a host cell, the method comprising the steps of: a) providing for each gene of the plurality of genes, a DNA fragment comprising an expression cassette for expression of the gene in the host cell, wherein the DNA fragment has staggered 5' and 3' ends that are part of a restriction enzyme recognition sequence, which recognition sequence is not present elsewhere in the DNA fragment and which recognition sequence at the 5' end of the DNA fragment differs from the recognition sequence at the 3' end of the DNA fragment, and wherein the overhang of the restriction enzyme recognition sequence at the 5' end is complementary with the overhang of the restriction enzyme recognition sequence at the 3' end; and b) combining the DNA fragments as obtained in a) by insertion of the DNA fragments into a unique restriction enzyme recognition site in a suitable vector, wherein the unique restriction enzyme recognition site in the vector has the same sequence as one of the two restriction recognition sequences at the 5' and 3' ends of the fragments in a), and wherein combination of a DNA fragment with a further DNA fragment produces a dysfunctional restriction enzyme site at the fusion site of the fragments. Preferably, the method of the invention is a method wherein the restriction enzyme recognition sequence at the 5' end of the DNA fragment and the restriction enzyme recognition sequence at the 3' end of the DNA fragment are recognition sequences of a restriction enzyme pair that provides complementary overhangs selected from the restriction enzyme pairs listed in Table 1.

A preferred embodiment of the invention is a method wherein for at least two genes in the plurality of genes the DNA fragments comprising the expression cassettes are assembled from subfragments comprising types of elements that together form a functional expression cassette, wherein the subfragments at least comprise in a 5' to 3' order: i) a subfragment comprising an upstream regulatory type of element; ii) a subfragment comprising a coding sequence type of element; and, iii) optionally, a subfragment comprising a downstream regulatory type of element; wherein the subfragments have staggered 5' and 3' ends, the overhang at the 5' end of each subfragment differs from the overhang at its 3' end, and the overhang at the 3' end of the subfragments comprising one type of element is complementary with the overhang at the 5' end of the subfragments comprising another type of element to allow assembly and operable linkage of the subfragments in a 5' to 3' order to form the functional expression cassette, or wherein, preferably, for all genes of the plurality of genes the DNA fragments comprising the expression cassettes are assembled from subfragments comprising types of elements that together form a functional expression cassette. Preferably, in this embodiment, the staggered 5' and 3' ends are part of recognition sequences for restriction enzymes, which recognition sequences are not present elsewhere in the subfragment, preferably which recognition sequences also are not present in the DNA fragment comprising the expression cassette and/or in the vector.. More preferably in this embodiment, the upstream regulatory type of element comprises at least a promoter and optionally further transcriptional and translational regulatory sequences, and the downstream regulatory type of element comprises at least one of a transcription termination sequence and a polyadenylation signal and optionally further regulatory sequences. Further preferred in this embodiment, at least one of the subfragments comprising an upstream regulatory type of element and the subfragments comprising a downstream regulatory type of element are provided as an E. *coli* library.

In the methods as described herein, the vector preferably is a cosmid vector, more preferably the vector is a binary vector system comprising a plurality of plasmid vectors and a cosmid vector, each plasmid vector comprising a plurality of 2 to 5 genes, each gene being a DNA fragment comprising an expression cassette, preferably wherein the plurality of genes present in the cosmid vector is assembled from each of the plurality of genes present in each of the plurality of plasmid vectors. Further preferred in the methods, the plurality of genes constitutes at least part of a metabolic pathway, wherein, more preferably, the plurality of genes comprise additional gene copies of at least part of the metabolic pathway, modified genes of at least part of the metabolic pathway and/or genes of at least part of a metabolic pathway that does not natively occur in the host cell.

In another aspect the invention pertains to a method for introducing a plurality of genes into a host cell in a single step, the method comprising the step of transforming the host cell with a vector obtained or obtainable by the above methods, and, optionally, isolating a host cell comprising the plurality of genes. Preferably in this method, the host cell is a microbial host, preferably a eukaryotic microbial host cell, more preferably a fungal host cell, most preferably a filamentous fungal host cell.

In further aspects the invention relates to vectors and host cells obtainable by the above methods.

In a final aspect the invention relates to methods for producing a compound of interest using the vectors and host cells as described herein. Thus, in one embodiment a compound of interest is produced in a method comprising the steps of culturing a host cell obtainable according to the methods as described herein, wherein the host cell is cultured under conditions conducive to the production of the compound of interest, and, optionally, the compound of interest is recovered. In another embodiment the method comprises the steps of: a) introducing a plurality of genes into the host cell in accordance with the above methods, whereby the plurality of genes constitute at least part of a metabolic pathway involved in the synthesis of the compound of interest; b) culturing the host cell obtained in a) under conditions conducive to the production of the compound of interest; and, c) optionally, recovering the compound of interest.

### Description of the invention

The present invention provides for a novel method for producing a vector for introducing a plurality of genes into a host cell in a single step. The vector (system) as described herein is a vector (system), in which it is possible to design novel pathways composed of any element of choice, such as any promoter and any coding sequence, and which advantageously allows the introduction of a plurality of genes or large biosynthetic pathways into the genome of a host cell, such as e.g. a filamentous fungus, in a single step. For instance, in this approach it is possible to conveniently increase the production level of a metabolite of choice and/or to construct a novel metabolic pathway in a filamentous fungus.

Thus, in a first aspect, there is provided a method for producing a vector for introducing a plurality of genes into a host cell.

The method preferably comprises the steps of: a) providing for at least a part of the genes of the plurality of genes, preferably for each gene of the plurality of genes, a DNA fragment comprising an expression cassette for expression of the gene in the host cell. Preferably, the DNA fragment comprising the expression cassette has staggered 5' and 3' ends that are part of a restriction enzyme recognition sequence. The 5' and 3' recognition sequences are not present elsewhere in the DNA fragment, and, preferably, are different from each other. The overhang of the restriction enzyme recognition sequence at the 5' end of the fragment further is preferably at least partially complementary with, or more preferably ligation-compatible and/or fully complementary with, the overhang of the restriction enzyme recognition sequence at the 3' end of the fragment.

The method preferably further comprises a step b) comprising: combining the DNA fragments as obtained in step a), i.e. combining the genes, by insertion of the fragments into a unique restriction enzyme recognition site in a suitable vector, wherein the unique recognition site in the vector has the same sequence as one of the two restriction enzyme recognition sequences at the 5' and 3' ends of the fragments in a). The complementary overhangs of the restriction enzyme recognition sequences at the respective 5' and 3' ends of the fragments allow annealing of the fragments due to the (at least partial) complementarity of the overhangs and subsequent covalent linking of the fragments by means of, for example, ligation, optionally in combination with DNA polymerase activity. The restriction enzyme recognition sequences at the 5' and 3' ends of the fragments being different from each other but providing complementary overhangs allows formation of a hybrid of the two recognition sequences at the site of fusion of the two DNA fragments, which hybrid sequence can no longer be digested (cut) by either of the original two restriction enzymes. Such a hybrid of two different recognition sequences is also referred to as a "dysfunctional restriction enzyme site" herein. Preferably, the unique recognition site in the vector is used for repetitive insertion of the DNA fragment comprising an expression cassette, i.e. the gene, whereby each insertion step provides a dysfunctional restriction enzyme site in between two DNA fragments and restores the unique recognition site in the vector to allow further insertion of a DNA fragment. In this way, DNA fragments comprising an expression cassette, i.e. genes, can be combined in a step-by-step fashion.

Preferably, the restriction enzyme recognition sequence at the 5' end of the DNA fragment and the restriction enzyme recognition sequence at the 3' end of the DNA fragment that are recognition sequences of a restriction enzyme pair that provides complementary overhangs when cut and a dysfunctional restriction enzyme site when ligated are selected from the restriction enzyme pairs listed in Table 1 below.

**Table 1**

| **Enzyme** | **Recognition sequences** | **Compatible enzymes for pair** |
|---|---|---|
| BamHI | G/GATCC | BclI, BglII, XhoII |
| BclI | T/GATCA | BamHI, BglII, XhoII |
| BglII | A/GATCT | BamHI, BclI, XhoII |
| EcoRI | G/AATTC | MunI |
| MunI | C/AATTG | EcoRI |
| NheI | G/CTAGC | SpeI, XbaI |
| NotI | GC/GGCCGC | XmaIII |
| NsiI | ATGCA/T | PstI |
| PstI | CTGCA/G | NsiI |
| SalI | G/CAGCT | XhoI |
| SpeI | A/CTAGT | AvrII, NheI, XbaI |
| XbaI | T/CTAGC | NheI, SpeI |
| XhoI | C/TCGAG | SalI |
| XmaIII | C/CCGGG | NotI |

A preferred pair is the pair BglII/BamHI.

An expression cassette for expression of a gene in the host cell is understood herein to mean a DNA fragment comprising all the sequence elements that are necessary for obtaining expression of the gene in the host cell in question, i.e. all necessary regulatory sequences operably linked to a coding sequence to be expressed. The regulatory sequence elements that are necessary for obtaining expression of the gene in the host cell in question will thus usually at least comprise regulatory elements for initiating transcription and translation of the coding sequence and preferably also regulatory elements for terminating transcription and translation of the coding sequence and, optionally for polyadenylation of the transcript, if relevant.

In a preferred embodiment of the method, for at least two genes of the plurality of genes, but more preferably for all the genes of the plurality of genes, the DNA fragments comprising an expression cassette, i.e. the genes, are assembled from subfragments comprising types of elements that together form a functional expression cassette. The DNA fragment comprising the expression cassette is assembled from subfragments comprising, in a 5' to 3' order: i) a subfragment comprising an upstream regulatory type of element; ii) a subfragment comprising a coding sequence type of element; and, iii) optionally, a subfragment comprising a downstream regulatory type of element. The subfragments each have staggered 5' and 3' ends, wherein the overhang at the 5' end of a subfragment differs from the overhang at its 3' end, the overhang at the 3' end of a subfragment comprising one type of element is preferably at least partially complementary with, or more preferably ligation-compatible and/or fully complementary with the overhang at the 5' end of a subfragment comprising another type of element to allow assembly and operable linkage of the subfragments in a 5' to 3' order to form the DNA fragment comprising the functional expression cassette, i.e. the gene. The subfragments comprising the type of elements as described above are conveniently assembled by annealing and ligating the respective subfragments. Such assembly may be done for at least two genes of the plurality of genes, preferably for all genes of the plurality of genes. Preferably, the staggered 5' and 3' ends are part of recognition sequences for restriction enzymes, which recognition sequences, preferably, are not present elsewhere in the subfragment. More preferably, the recognition sequences for restriction enzymes are also not present in the vector and/or in the DNA fragment comprising the expression cassette. Such absence of the 5' and 3' recognition sequences conveniently allows insertion of the subfragments into the vector in a one-step reaction and exchange of one type of element for a different type of element having an equivalent function. In addition, the restriction enzyme recognition sequences may be chosen for containing functional sequences or parts thereof, for instance a translation start site.

The regulatory type of elements typically are chosen from elements that are necessary to obtain expression of the gene in the host cell of choice. The upstream regulatory type of element will usually at least comprise a promoter. Optionally, the upstream regulatory type of element may comprise further transcriptional and translational regulatory sequences, including e.g. enhancers. The upstream regulatory type of element, when operably linked to the type of element comprising the coding sequence, will ensure that the coding sequence is transcribed and preferably also translated in the host cell in question. The downstream regulatory type of element will usually comprises at least a transcription termination sequence and preferably, if relevant, also a polyadenylation signal and may comprise further regulatory sequences.

Preferably, at least three type of elements are used to assemble a functional expression cassette: an upstream type element, an element comprising the coding sequence (of the gene to be expressed), and a downstream type element.

The operable linkage of the subfragments comprising the functional elements being part of the functional expression cassette and the insertion in a vector may advantageously be done in a one-step reaction, whereas the combination of genes being part of the plurality of genes in a vector may advantageously be done iteratively, by the step-by-step insertion of a DNA fragment comprising an expression cassette. In the same way, a cosmid vector can be build up by step-by-step insertion of DNA fragments, each fragment containing a plurality of 2 to 5 assembled genes, in the unique restriction enzyme site of the cosmid vector.

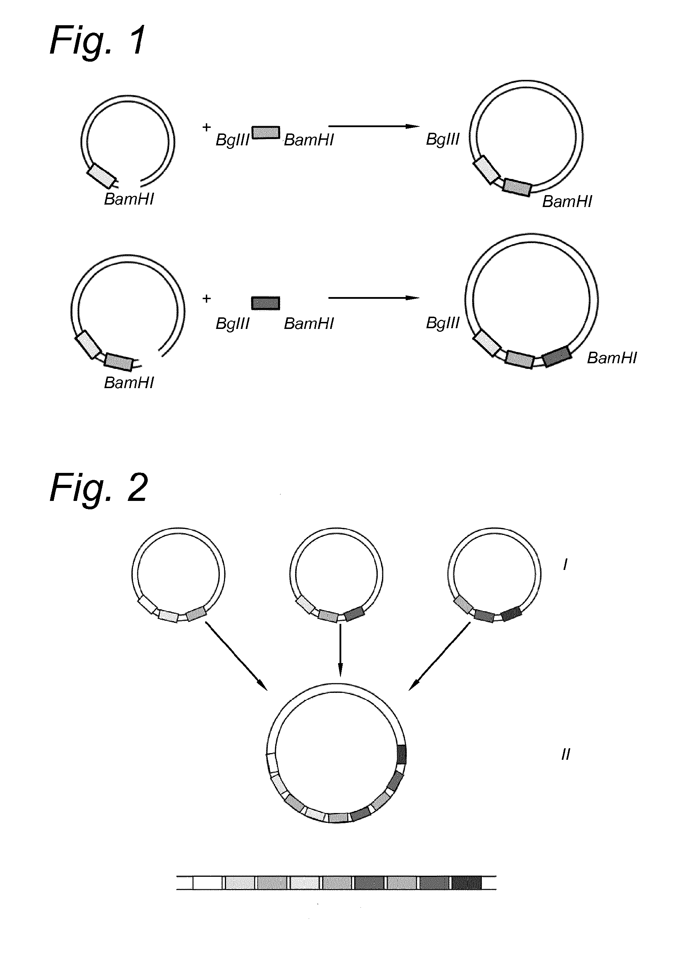

The construction of plasmid and cosmid vectors as described hereinabove is schematically outlined in Figures 1 and 2, respectively.

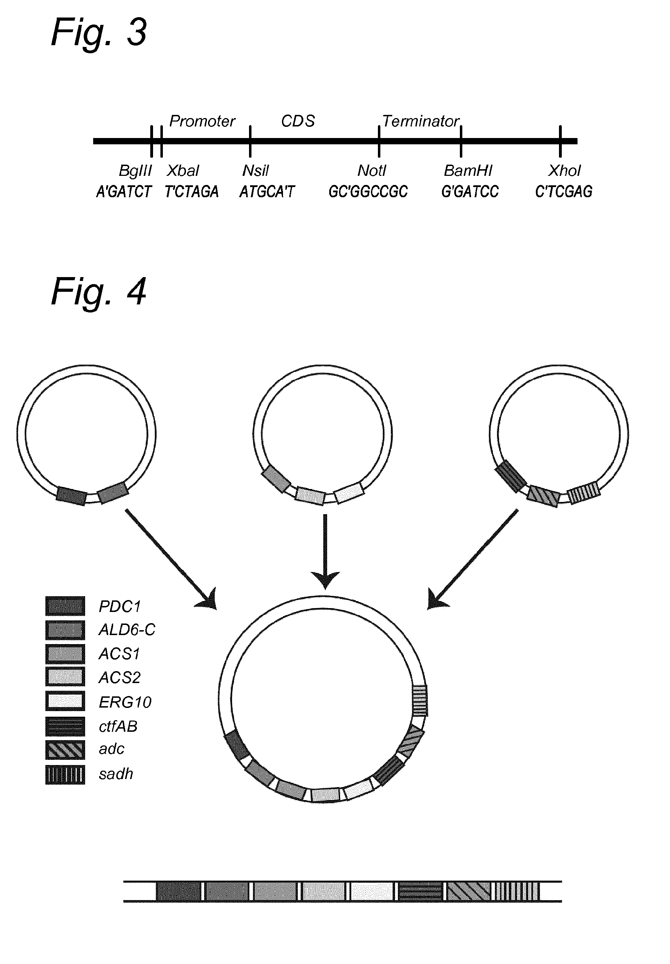

Preferred restriction enzyme sites (in a 5' to 3' order) flanking (in a 5' to 3' order) an upstream or promoter element are BglII and NsiI, preferred restriction enzyme sites flanking (in a 5' to 3' order) a coding sequence are NsiI and NotI, and preferred restriction sites flanking (in a 5' to 3' order) a downstream or terminator element are NotI and BamHI. This implicates that the assembled DNA fragment comprising the expression cassette, i.e. the assembled gene, is flanked by a BglII site and a BamHI site (see Figure 3). The XhoI site as indicated in Figure 3 may be used for insertion of a selection marker.

The DNA fragments and subfragments to be used in the methods of the invention may conveniently be provided at least in part by custom DNA synthesis and by methods known in the art per se. The elements are synthesized, and optionally modified, not to contain any of the restriction enzyme sites mentioned to flank and to operably link the elements. To that end, mutations may be introduced in the elements where appropriate, including silent mutations in the coding sequence. To increase the likelihood that a coding sequence is expressed at sufficient levels and in active form in the transformed host cells, the coding sequence may be adapted to optimize its codon usage to that of the host cell in question. Each synthesized subfragment may conveniently be cloned in an *E. coli* vector. In this way, an *E. coli* library of subfragments comprising functional elements is provided. Such a library is especially convenient for at least one of the subfragments comprising an upstream regulatory type of element and the subfragments comprising a downstream regulatory type of element.

The operable linkage of subfragments comprising functional elements provides an assembled DNA fragment comprising an expression cassette, i.e. an assembled gene, whereas the linkage of assembled genes provides a large fragment comprising a plurality of assembled genes. Preferably, the plurality of assembled genes constitutes, for instance, at least part of a metabolic pathway, preferably a complete metabolic pathway.

By way of the functional elements, the assembled genes and the plurality of assembled genes being "standardized" at their ends, i.e. by having staggered ends as described herein, the functional elements and assembled genes do not occur in nature or natively in the host cell in question.

A "plurality of genes", as used herein, refers to two or more genes. Advantageously, the method as described herein is applicable to the single step transformation into a filamentous fungus of at least 5, 10, 20, 21, 22, 23, 24, 25, 26, 27, 28, 29, 30, 40, 50 or more genes using a single vector.

As used herein, the terms "gene" and "expression cassette" refer to a DNA fragment comprising a transcribed region comprising a coding sequence, which is transcribed into an RNA molecule (e.g. an mRNA) in a cell, which mRNA is capable of being translated into a protein or peptide, and suitable regulatory regions (e.g. a promoter and terminator) operably linked thereto. A gene will usually comprise several operably linked elements, such as a promoter, a 5' untranslated leader sequence, a coding sequence and a terminator.

As used herein, the term "promoter" refers to a nucleic acid fragment that functions to initiate and control the transcription of one or more coding sequences, and is structurally identified by the presence of a binding site for DNA-dependent RNA polymerase, transcription initiation sites and any other DNA sequences, including, but not limited to transcription factor binding sites, repressor and activator protein binding sites, and any other nucleotide sequences known to one of skill in the art to act directly or indirectly to regulate the transcription from the promoter. A "constitutive" promoter is a promoter that is always active in most cells under most physiological and developmental conditions. An "inducible" promoter is a promoter that is physiologically or developmentally regulated, and can be regulated e.g. by the application of a chemical inducer.

A promoter for expression of the coding sequence as described herein in a host cell may be any promoter that functions in the host cell in question, and includes promoters that are insensitive to catabolite (glucose) repression. Promoters having these characteristics are widely available and known to the skilled person. Suitable examples of such promoters for filamentous fungi include e.g. promoters from glycolytic genes such as the phosphofructokinase *(PFK),* triose phosphate isomerase *(TPI),* glyceraldehyde-3-phosphate dehydrogenase *(GPD, TDH3* or *GAPDH),* pyruvate kinase *(PKI),* phosphoglycerate kinase *(PGK),* glucose-6- phosphate isomerase promoter *(PGI1).* Other useful promoters are ribosomal protein encoding gene promoters, the lactase gene promoter *(LAC4),* alcohol dehydrogenase promoters *(ADH1, ADH4,* and the like), the enolase promoter *(ENO),* the hexose(glucose) transporter promoter *(HXT7),* and the cytochrome cI promoter *(CYC1).* Other promoters, both constitutive and inducible, and enhancers or upstream activating sequences will be known to those of skill in the art. The promoter that is operably linked to a coding sequence as defined above may be the promoter natively coupled to the coding sequence or may be a promoter not natively coupled thereto (heterologous). Preferably, the promoter is homologous to the fungal host cell.

The 5' untranslated leader sequence (also referred to as 5'UTR), which corresponds to the transcribed mRNA sequence upstream of the translation start codon and comprises e.g. sequences involved in translation initiation, may be included in the promoter element, in the coding sequence element, or may be provided as a separate type of element. Preferably, the 5'UTR is included in the promoter element.

As used herein, the term "terminator" refers to a nucleic acid fragment that functions to terminate transcription and is recognized by protein factors involved in such termination. It typically contains a polyadenylation site.

As used herein, the term "operably linked" or "operable linkage" refers to a linkage of polynucleotide elements in a functional relationship. A polynucleotide element is "operably linked" to another polynucleotide element when it is placed into a functional relationship with the other polynucleotide element. For instance, a promoter sequence is operably linked to a coding sequence if it affects initiation and control of the transcription of the coding sequence. Operably linked means that the DNA sequences being linked are typically contiguous and, when joining two protein encoding sequences, are in the same reading frame.

The vector for insertion of the plurality of genes may be any vector that is suitable for introducing the plurality of genes into a host cell of choice. The vector may be a plasmid or a cosmid vector, depending on the size of the fragment comprising the plurality of genes. For a plasmid vector, the maximum size typically will be 12-15 kb, whereas for a cosmid vector this size is about 45 kb.

In one embodiment, one or more plasmid vectors may advantageously be used as an intermediate in the construction of a cosmid vector. The plasmid vectors may be used for the step-by-step insertion of assembled genes, and, where necessary, for the assembly of genes from their functional elements, to provide a plurality of plasmid vectors each containing a plurality of up to about five genes. The cosmid vector may be build up by the step-by-step insertion of each of the plurality of genes as present in each of the plurality of plasmid vectors.

The method as described in this aspect thus advantageously allows for multiple and easy insertion of a plurality of genes in one vector, also in a vector capable of carrying large DNA fragments, such as a cosmid vector.

In a second aspect, there is provided a vector for transformation of a host cell, such as e.g. a filamentous fungus, the vector comprising a plurality of genes, which plurality of genes is assembled as described in the first aspect. The vector is preferably obtained or obtainable by any of the methods as described in the first aspect. Preferably, there is provided a binary vector system comprising a plurality of plasmid vectors and a cosmid vector, the plasmid vectors advantageously being used as an intermediate in the construction of the cosmid vector, as also described in the first aspect.

In a further aspect, there is provided a method for introducing a plurality of genes into a host cell. The method preferably comprises the steps of transforming a host cell with a vector comprising the plurality of genes, which vector is obtained or obtainable by any of the methods as described in the first aspect, and screening for or selecting transformed host cells containing the plurality of genes. Optionally, the method can comprise isolating the host cell comprising the plurality of genes. The host cell preferably is a microbial host cell, preferably a eukaryotic microbial host cell, more preferably a fungal host cell, most preferably a filamentous fungal host cell.

Preferably the method is a method for introducing a plurality of genes into a host cell in a single transformation step. This is especially advantageous when aiming at the introduction of a plurality of genes into a filamentous fungus.

The vector may be any vector which is suitable for transformation of the host cell in question, i.e. a vector that can be selected in the host cell, such as e.g. a filamentous fungus.

The plurality of genes to be introduced in a host cell preferably constitutes at least part of a metabolic pathway, e.g. a biosynthetic pathway, more preferably constitutes a complete metabolic or biosynthetic pathway, for instance a metabolic or biosynthetic pathway directing the synthesis of a compound of interest to be produced by the host cell. The plurality of genes can comprise additional gene copies of at least part of the metabolic pathway, modified genes of at least part of the metabolic pathway and/or genes of at least part of a metabolic pathway that does not natively occur in the host cell. To increase the likelihood that a coding sequence is expressed at sufficient levels and in active form in the transformed host cells, the coding sequence may be adapted to optimize its codon usage to that of the host cell in question.

The microbial host cell preferably is a filamentous fungus host cell. Filamentous fungi are herein defined as eukaryotic microorganisms that include all filamentous forms of the subdivision Eumycotina. These fungi are characterized by a vegetative mycelium composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism of most filamentous fungi is obligate aerobic. Preferred filamentous fungi as host cells belong to the genera *Aspergillus, Monascus, Trichoderma, Humicola, Acremonium, Fusarium,* and *Penicillium.*

In addition to the plurality of assembled genes, the vector may contain a selection marker for the selection and isolation of transformed host cells. The term "selection marker" is a term familiar to one of ordinary skill in the art and is used herein to describe any genetic entity which, when expressed, can be used to select for a cell or cells containing the selection marker. A variety of selectable marker genes are available for use in the transformation of fungi. Suitable markers include auxotrophic marker genes involved in amino acid or nucleotide metabolism, such as e.g. genes encoding ornithine-transcarbamylases *(argB),* orotidine-5'-phosphate decarboxylases (*pyrA* or *pyrG*)*,* or involved in carbon or nitrogen metabolism, such as e.g. nitrate reductase *(niaD),* and antibiotic resistance markers such as genes providing resistance against hygromycin, phleomycin, bleomycin or neomycin (G418). The transformation marker may be flanked by a XhoI, AvaI, PaeR71 or SalI restriction enzyme site.

The vector may provide integration of the pathway in the genome of the host cell or may provide extrachromosomal replication of the vector. Preferably, the vector provides for integration in the genome.

For transformation of a fungus with a relatively small pathway (up to about 15 kb, e.g. from 2 to about 5 genes), typically a plasmid vector is used.

For transformation of a fungus with a larger pathway (up to about 45 kb, e.g. from 6 up to about 50 genes), a cosmid vector may be used.

Transformation protocols for transformation of filamentous fungi are commonly known in the art. The transformation of *A. niger* protoplasts using cosmid vectors may, for instance, be done as described by Yelton, Timberlake, & van den Hondel, 1985 and Yelton & Hamer, 1984 for *Aspergillus nidulans,* which method has been applied for many filamentous fungi, see e.g. Hynes, 1996.

In still a further aspect, there is provided a host cell comprising the vector as described in the previous aspects.

In still a further aspect, there is provided a method for producing a compound of interest using the host cell from the previous aspect. Cells are cultured under conditions conducive to production of the compound of interest, and, optionally, the compound of interest is recovered from the culture broth. In particular, the method for producing the compound of interest comprises the introduction of a plurality of genes into the host cell using the method as described in the first aspect, the plurality of genes encompassing at least part of the biosynthetic pathway directing synthesis of the compound of interest. The culturing of the cells and the recovery of the compound of interest from the culture broth may be done using commonly known procedures.

The invention advantageously allows for the introduction into a microbial genome, preferably into a fungal genome, by a single transformation step, of a plurality of genes constituting at least part of a metabolic pathway, preferably constituting a complete metabolic pathway. The genes constituting at least part of a metabolic pathway may encompass additional gene copies of at least part of the metabolic pathway, modified genes of at least part of the metabolic pathway and/or genes of at least part of a metabolic pathway that does not natively occur in the fungus in question.

Examples of metabolic pathways are the itaconic acid biosynthetic pathway from *Aspergillus terreus* consisting of the genes *cadA, mttA* and *mfsA,* and the lovastatin biosynthetic pathway from *Aspergillus terreus* consisting of the genes *lovA, lovB, lovC, lovD, loveE* and *lovF.* An example of a metabolic pathway consisting of genes from different organisms is the pathway providing production of isopropanol, consisting of the *PDC1, ALD6-C, ACS1, ACS2* and *ERG10* genes from *Candida utilis,* the *ctfA* and *ctfB* genes from *Clostridium acetobutylicum* and the *adc* and *sadh* genes from *Clostridium beijerinckii.*

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

### Description of the figures

Figure 1 schematically shows the construction of a plasmid vector containing 3 assembled genes.
Figure 2 schematically shows the construction of a cosmid vector containing 3 clusters each comprising 3 assembled genes.
Figure 3 shows an example of a gene assembled from its functional elements and the restriction enzyme sites used for assembly.
Figure 4 schematically shows the construction of an isopropanol biosynthesis pathway in a cosmid vector.

### Examples

All plasmids are amplified in *E.coli* DH5α and purified using the Macherey-nagel Nucleobond extra® plasmid purification kit according to the manufacturers instructions.

All fragments are isolated from 1.5% agarose gel using the Thermo Scientific GeneJET Gel Extraction kit according to the manufacturers instructions.

In the examples provided herein, the term "funbrick" refers to a standardized functional element, a standardized assembled gene or a plurality of standardized assembled genes, as will be apparent from the context wherein the term is used.

### Example 1 Construction of the Funbrick® basic plasmid vector

The standard "Funbrick" used for assembly as depicted in Figure 1 is constructed as a pUC19-derived vector. The pUC19-derived vector contains a synthetic DNA insert in the multiple cloning site that consists of a modified *xlnD* promoter, SEQ ID NO: 1, from *A. niger* flanked by the restriction sites XbaI and NsiI, a non-coding part flanked by restriction sites NsiI and NotI and the *xlnD* terminator, SEQ ID NO: 2, from *A. niger* flanked by NotI and BamHI. The XhoI and the BglII sites are created using a QuikChange Lightning Site-Directed mutagenesis kit according to the manufacturers instructions. For the creation of the BglII site upstream the XbaI site primers LS_BglII and LS_BglII_antisense are used. The XhoI site is created using primers LS_XhoI and LS_XhoI_antisense.

Primers:
LS_BglII (5'-agcttgcatgcctgcagatctactctagacgaatgagg-3') (SEQ ID NO: 3)
LS_BglII_antisense (5'-cctcattcgtctagagtagatctgcaggcatgcaagct-3') (SEQ ID NO: 4)
LS_XhoI (5'- gagacgaaagggcctcgagatacgcctatttttat-3') (SEQ ID NO: 5)
LS_XhoI_antisense (5'-ataaaaataggcgtatctcgaggccctttcgtctc-3') (SEQ ID NO: 6)

### Example 2 Construction of the mttA Funbrick

The *mttA* Funbrick was constructed by digesting the Funbrick basic vector with NsiI and NotI according the manufacturers instructions. The *Aspergillus terreus mttA* gene, as described as ORF 14 by Kennedy et al (Kennedy, 1999) was codon-optimized synthesized (SEQ ID NO: 7) and subcloned by DNA 2.0 (Menlo Park, CA , USA) in the vector pORF14. The cds of *mttA* was digested with NsiI and NotI and ligated into the basic Funbrick vector resulting in pFB001.

### Example 3 Insertion of a fungal transformation marker in pFB001

For the insertion of a fungal transformation marker pFB001 was amplified in *E. coli* and purified using the plasmid purification kit according to the manufacturers instructions. The *A. niger pyrA* was synthesized (SEQ ID NO: 8) by Baseclear (Leiden, The Netherlands) and cloned in the XhoI site of the previously described pFB001 resulting in plasmid pFB107.

### Example 4 Construction of the mfsA Funbrick

The *mfsA* Funbrick was constructed by digesting the Funbrick basic vector with NsiI and NotI according the manufacturers instructions. The *Aspergillus terreus mfsA* gene, described as ORF 16 by Kennedy et al (Kennedy, 1999) was codon-optimized synthesized (SEQ ID NO: 9) and subcloned by DNA 2.0 (Menlo Park, CA , USA) in the vector pORF16. The cds of *mfsA* was digested with NsiI and NotI and ligated into the basic Funbrick pFB001 resulting in pFB003.

### Example 5 Construction of the cadA Funbrick

The *cadA* Funbrick was constructed by digesting the Funbrick basic vector with NsiI and NotI according the manufacturers instructions. The *Aspergillus terreus cadA* gene, as described as ORF 15 by Kennedy et al was codon-optimized synthesized (SEQ ID NO: 10) and subcloned by DNA 2.0 (Menlo Park, CA , USA) in the vector pCAD. The cds of *cadA* was digested with NsiI and NotI and ligated into the basic Funbrick pFB001 resulting in pFB004.

The gene was identified as encoding *cis*-aconitate decarboxylase as described in patent application WO2009102205.

### Example 6 Construction of the Funbrick containing the A. terreus itaconic acid biosynthetic pathway

The Funbrick pFB002 containing the *mttA* cds was digested with BglII and BamHI according the manufacturers instructions. The resulting fragment was isolated and ligated in the BamHI digested pFB004 resulting in the plasmid pFB005.

The Funbrick pFB003 was digested with BglII and BamHI according the manufacturers instructions. The resulting fragment containing the *mfsA* was isolated and ligated in the BamHI digested pFB005 resulting in the plasmid pFB006.

### Example 7 Construction of the ympc Funbricks

The three S. *cerevisiae* mitochondrial pyruvate carrier subunits, ympcA (SEQ ID NO: 11), ympcB (SEQ ID NO: 12) and ympcC (SEQ ID NO: 13), as described by Herzig et al 2012 (Herzig et al., 2012) were codon-optimized synthesized and subcloned by Baseclear in the vector pUC57. The sequences were released from the vectors by digestion with NsiI and NotI and ligated into the FB001, which had been digested with NsiI and NotI. This resulted in vectors pFBympc1-M, pFBympc2-M and pFBympc3-M.

A vector was then constructed by ligation of pFBympc1-M digested with BamHI and the smaller fragments of pFBympc2-M digested with BglII and BamHI, resulting in the vector pFBympc12-M. A fungal transformation marker was inserted by constructing a vector by ligation of the 2357bp fragment of pGW635 digested with XhoI and the pFBympc12-M linearized by digestion with XhoI, resulting in vector pFBympc12.

### Example 8 Construction of the fmpc Funbricks

The two *A. niger* mitochondrial pyruvate carrier homologues fmpcA (SEQ ID NO: 14) and fmpcB (SEQ ID NO: 15) that were identified by sequence alignment using *Saccharomyces cerivisiae* MPC1, MPC2 and MPC3 (SEQ ID NO: 16, 17 and 18)) and standard BLAST settings, were codon-optimized synthesized and subcloned by Baseclear in the vector pUC57. The sequences were released from the vectors by digestion with NsiI and NotI and ligated into the FB001, which had been digested with NsiI and NotI. This resulted in vectors pFBfmpcA-M and pFBfmpcB-M.

A vector was then constructed by ligation of pFBfmpcA-M digested with BamHI and the smaller fragment of pFBfmpcB-M digested with BglII and BamHI, resulting in the vector pFBfmpcAB-M. A fungal transformation marker was inserted by constructing a vector by ligation of the 2357bp fragment of pGW635 digested with XhoI and the pFBfmpcAB-M linearized by digestion with XhoI, resulting in vector pFBfmpcAB.

### Example 9 Construction of the pFB201 Funbrick

The pFB201 Funbrick was constructed by digesting the Funbrick basic vector with NsiI and NotI according the manufacturers instructions. The *Rhizopus oryzae* malate dehydrogenase coding gene *RoMDH* (Xu, Liu, & Chen, 2012) was codon-optimized synthesized (SEQ ID NO: 19) and subcloned by Baseclear (Leiden, The Netherlands) in the Funbrick basic vector resulting in pFB201.

### Example 10 Construction of the pFB202 Funbrick

The pFB202 Funbrick was constructed by digesting the Funbrick basic vector with NsiI and NotI according the manufacturers instructions. The *Aspergillus niger icl* gene (Meijer et al., 2009) was codon-optimized synthesized (SEQ ID NO: 20) and subcloned by Baseclear (Leiden, The Netherlands) in the Funbrick basic vector resulting in pFB202.

### Example 11 Construction of the pFB203 Funbrick

The pFB203 Funbrick was constructed by digesting the Funbrick basic vector with NsiI and NotI according the manufacturers instructions. The *Saccharomyces cerevisiae* succinate/fumarate mitochondrial carrier coding gene ACR1 (also called SFC1) (L. Palmieri, Lasorsa, De Palma, & Palmieri, 1997) was codon-optimized synthesized (SEQ ID NO: 21) and subcloned by Baseclear (Leiden, The Netherlands) in the Funbrick basic vector resulting in pFB203.

### Example 12 Construction of the pFB204 Funbrick

The pFB204 Funbrick was constructed by digesting the pFB107 vector with NsiI and NotI according the manufacturers instructions. The *Arabidopsis thaliana FUM1* gene (without its mitochondrial signal coding sequence) (Pracharoenwattana et al., 2010) was amplified by PCR with the oligonucleotides: JT-AtFUMITr-FW (SEQ ID NO: 22): CCATGCATTCGACCTCGTTTAGGGA and JT-AtFUM1-RV (SEQ ID NO: 23): ATGCGGCCGCTCAATCGGAGGGACCAA, using cDNA prepared from total RNA isolated from *Arabidopsis thaliana* leaves as a template, and subcloned as FUM1 (SEQ ID NO: 24) in the pFB002 resulting in pFB204.

### Example 13 Construction of the pFB205 Funbrick

The pFB204 Funbrick was constructed by digesting the pFB107 vector with NsiI and NotI according the manufacturers instructions. The *Arabidopsis thaliana FUM2* gene [4] was amplified by PCR with the oligonucleotides:
JT-FumR2ArabidT-FW (SEQ ID NO: 25):
   ATATGCATGCTTTGACAATGCAGTTTG and
   JT-FumR2ArabidT-RV (SEQ ID NO: 26):
GCGGCCGCTTAATCAGATGGACCAATC, using a cDNA prepared from total RNA isolated from *Arabidopsis thaliana* leaves as a template, and subcloned as FUM2 (SEQ ID NO: 27) in the pFB002 resulting in pFB204.

### Example 14 Construction of the pFB206 Funbrick

The pFB206 Funbrick was constructed by digesting the pFB107 vector with NsiI and NotI according the manufacturers instructions. The *Aspergillus niger fumA* gene (without its mitochondrial signal coding sequence) was amplified by PCR with the oligonucleotides:
JT-FumR-Tr-A.niger-FW (SEQ ID NO: 28):
   CCATGCATGAGACCGATGCCTTCGG and
JT-FumR-A.niger-RV (SEQ ID NO: 29): ATGCGGCCGCTTATTTCTTCTCCTTGG using a gDNA preparation from *Aspergillus niger* N593 strain as a template, and subcloned as FUMA (SEQ ID NO: 30) in the pFB107 resulting in pFB206.

### Example 15 Construction of the pFumRs

The pFumRs Funbrick was constructed by digesting a pUC19-derived vector (containing a multiple cloning site that consists of a modified *xlnD* promoter, SEQ ID NO: 31, *from A. niger* flanked by the restriction sites XbaI and NsiI, a non-coding part flanked by restriction sites NsiI and NotI and the *xlnD* terminator, SEQ ID NO: 2, from *A. niger* flanked by NotI and BamHI) with NsiI and NotI according the manufacturers instructions. The *Rhizopus oryzae FumR* gene was codon-optimized synthesized and subcloned by DNA 2.0 (USA) in the pUC19-derived vector resulting in pFumRs.

### Example 16 Construction of the pFB207 Funbrick

The pFB207 Funbrick was constructed by digesting the pFB107 vector with NsiI and NotI according the manufacturers instructions. The *Rhizopus orizae FumR* gene was obtained with an NsiI and NotI digestion from the plasmid pFumRs, and subcloned in the pFB107 resulting in pFB207.

### Example 17 Construction of the pFB401 Funbrick

The pFB401 Funbrick was constructed by digesting the pFB401 vector with NsiI and NotI according the manufacturers instructions. The *Rhizopus oryzae FumT* gene was codon-optimized synthesized (SEQ ID NO: 32) and subcloned by Baseclear (Leiden, The Netherlands) in the pFB107 vector resulting in pFB401.

### Example 18 Construction of the Fumarate biosynthesis pathway Funbrick

The Fumarate biosynthestic pathway Funbrik was constructed by:
1) Digesting the pFBFUMT vector with BamHI according the manufacturers instructions.
2) Ligating a BglII-BamHI fragment containing the expression cassette *xln*D*ₚ*::*cPYCs:: xlnD*ₜ (obtained as described in Example 2 of international patent application PCT/NL2013/050174 in the BamHI linearized pFBFUMT, resulting in pFBPYC+FUMT.
3) Digesting the pFBPYC+FUMT vector with BamHI according the manufacturers instructions.
4) Ligating a BglII-BamHI fragment containing the expression cassette *xlnD*p::*RoMDH*:: *xlnD*ₜ in the BamHI linearized pFBPYC+FUMT, resulting in pFBPYC+RoMDH+FUMT.
5) Digesting the pFBPYC+RoMDH+FUMT vector with BamHI according the manufacturers instructions.
6) Ligating a BglII-BamHI fragment containing the expression cassette *xlnD*p::*RoFUMR*:: *xlnD*ₜ in the BamHI linearized pFBPYC+RoMDH+FUMT, resulting in the Fumarate biosynthesis pathway Funbrick.

### Example 19 Funbrick cosmid vector

The Funbrick cosmid vector is constructed from the cosmid pLorist2 (Addgene) (SEQ ID NO: 33). In pLorist2 a SalI site was made at position 917 by in vitro mutagenesis resulting in pLorist2SalI (SEQ ID NO: 34) In this SalI site a synthetic fragment containing the *A. niger pyrA* gene was ligated to serve as a selection marker in transformation.

### Example 20 Construction of an isopropanol biosynthesis pathway

The construction of the full pathway in a cosmid vector is schematically shown in Figure 4.

The *ALD6-C* Funbrick was constructed by digesting the pFB107 Funbrick vector with NsiI and NotI according the manufacturers instructions. The *Candida utilis ALD6-C* gene, as described in Tamakawa et al was synthesized and subcloned by DNA 2.0 (Menlo Park, Calif, USA) in the pFB107 Funbrick vector resulting in pFB_ALD6-C. The *ACS1* Funbrick was constructed by digesting the pFB107 Funbrick vector with NsiI and NotI according the manufacturers instructions. The *Candida utilis ACS1* gene, as described in Tamakawa et al was synthesized (SEQ ID NO: 35) and subcloned by DNA 2.0 (Menlo Park, Calif, USA) in the pFB107 Funbrick vector resulting in pFB_ACS1.

The *ACS2* Funbrick was constructed by digesting the pFB107 Funbrick vector with NsiI and NotI according the manufacturers instructions. The *Candida utilis ACS2* gene, as described in Tamakawa et al was synthesized (SEQ ID NO: 36) and subcloned by DNA 2.0 (Menlo Park, Calif, USA) in the pFB107 Funbrick vector resulting in pFB_ACS2.

The *ERG10* Funbrick was constructed by digesting the pFB107 Funbrick vector with NsiI and NotI according the manufacturers instructions. The *Candida utilis ERG10* gene, as described in Tamakawa et al was synthesized (SEQ ID NO: 37) and subcloned by DNA 2.0 (Menlo Park, Calif, USA) in the pFB107 Funbrick vector resulting in pFB_ERG10.

The *ctfA* Funbrick was constructed by digesting the pFB107 Funbrick vector with NsiI and NotI according the manufacturers instructions. The *Clostridium acetobutylicum ctfA* gene, as described in Tamakawa et al was synthesized (SEQ ID NO: 38) and subcloned by DNA 2.0 (Menlo Park, Calif, USA) in the pFB107 Funbrick vector resulting in pFB_ctfA.

The *ctfB* Funbrick was constructed by digesting the pFB107 Funbrick vector with NsiI and NotI according the manufacturers instructions. The *Clostridium acetobutylicum ctfB* gene, as described in Tamakawa et al was synthesized (SEQ ID NO: 39) and subcloned by DNA 2.0 (Menlo Park, Calif, USA) in the pFB107 Funbrick vector resulting in pFB_ctfB.

The *adc* Funbrick was constructed by digesting the pFB107 Funbrick vector with NsiI and NotI according the manufacturers instructions. The *Clostridium beijerinckii adc* gene, as described in Tamakawa et al was synthesized (SEQ ID NO: 40) and subcloned by DNA 2.0 (Menlo Park, Calif, USA) in the pFB107 Funbrick vector resulting in pFB_adc.

The *sadh* Funbrick was constructed by digesting the pFB107 Funbrick vector with NsiI and NotI according the manufacturers instructions. The *Clostridium beijerinckii sadh* gene, as described in Tamakawa et al was synthesized (SEQ ID NO: 41) and subcloned by DNA 2.0 (Menlo Park, Calif, USA) in the pFB107 Funbrick vector resulting in pFB_sadh.

The *xlnD*ₚ::*ALD6-C:: xlnD*ₜ fragment is obtained by digesting the pFB_ALD6-C funbrick with BglII and BamHI and ligated into the BamHI digested pFB_PDC1 funbrick resulting in the pFB_PDC1+ALD6-C vector.

The *xlnD*ₚ::*ACS1*:: *xlnD*ₜ fragment is obtained by digesting the pFB_ACS1 funbrick with BglII and BamHI and ligated into the BamHI digested pFB_ACS2 funbrick resulting in the pFB_ACS1+ACS2 vector.

The *xlnD*ₚ::*ERG10*:: *xlnD*ₜ fragment is obtained by digesting the pFB_ERG10 funbrick with BglII and BamHI and ligated into the BamHI digested pFB_ACS2 funbrick resulting in the pFB_ACS1+ACS2+ERG10 vector.

The *xlnD*ₚ*::ctfA:: xlnD*ₜ fragment is obtained by digesting the pFB_ctfA funbrick with BglII and BamHI and ligated into the BamHI digested pFB_ctfB funbrick resulting in the pFB_ctfAB vector.

The *xlnD*ₚ::*adc:: xlnD*ₜ fragment is obtained by digesting the pFB_ADC funbrick with BglII and BamHI and ligated into the BamHI digested pFB_ctfAB funbrick resulting in the pFB_ctfAB+adc vector.

The *xlnD*ₚ::*sadh*:: *xlnD*ₜ fragment is obtained by digesting the pFB_sadh funbrick with BglII and BamHI and ligated into the BamHI digested pFB_ctfAB+adc funbrick resulting in the pFB_ctfAB+adc+sadh vector.

The *xlnD*ₚ::*PDC1*:: *xlnD*ₜ:: *xlnD*ₚ::*ALD6-C*:: *xlnD*ₜ fragment is obtained by digesting the pFB_PDC1+ALD6-C vector with BglII and BamHI and ligated into the BamHI digested Funbrick cosmid vector resulting in pFBC_PDC1+ALD6-C (Figure 4).

The *xlnD*ₚ*::ACS1:: xlnD*ₜ:: *xlnD*ₚ*::ACS2*:: *xlnD*ₜ:: *xlnD*ₚ::*ERG10*:: *xlnD*ₜ fragment is obtained by digesting the pFB_ACS1+ACS2+ERG10 vector with BglII and BamHI and ligated into the BamHI pFBC_PDC1+ALD6-C Funbrick cosmid vector resulting in pFBC_PDC1+ALD6-C+ACS1+ACS2+ERG10 (Figure 4).

The *xlnD*ₚ::*ctfAB*:: *xlnD*ₜ:: *xlnD*ₚ::*adc*:: *xlnD*ₜ:: *xlnD*ₚ::*sadh*:: *xlnD*ₜ fragment is obtained by digesting the pFB_ctfAB+adc+sadh vector with BglII and BamHI and ligated into the BamHI pFBC_PDC1+ALD6-C+ ACS1+ACS2+ERG10 Funbrick cosmid vector resulting in pFBC_PDC1+ALD6-C+ ACS1+ACS2+ERG10+ctfAB+adc+sadh (Figure 4).

### List of references

Herzig, S., Raemy, E., Montessuit, S., Veuthey, J.-L., Zamboni, N., Westermann, B., et al. (2012). Identification and functional expression of the mitochondrial pyruvate carrier. Science (New York, NY), 337(6090), 93-96. doi:10.1126/science.1218530
Hynes, M. J. (1996). Genetic transformation of filamentous fungi. Journal of Genetics, 75(3), 297-311. doi:10.1007/BF02966310
Kennedy, J. (1999). Modulation of Polyketide Synthase Activity by Accessory Proteins During Lovastatin Biosynthesis. Science (New York, NY), 284(5418), 1368-1372. doi:10.1126/science.284.5418.1368
Medema, M.H. et al., 2011. Exploiting plug-and-play synthetic biology for drug discovery and production in microorganisms. Nature reviews. Microbiology, 9(2), pp.131-137.
Meijer, S., Otero, J., Olivares, R., Andersen, M. R., Olsson, L., & Nielsen, J. (2009). Overexpression of isocitrate lyase -- Glyoxylate bypass influence on metabolism in Aspergillus niger. Metabolic engineering*.* doi:10.1016/j.ymben.2008.12.002
Orbach, M. J. (1994). A cosmid with a HyR marker for fungal library construction and screening. Gene, 2;150(1):159-62.
Palmieri, L., Lasorsa, F. M., De Palma, A., & Palmieri, F. (1997). Identification of the yeast ACR1 gene product as a succinate-fumarate transporter essential for growth on ethanol or acetate. FEBS Letters, 417(1), pp.114-118.
Pracharoenwattana, I., Zhou, W., Keech, O., Francisco, P. B., Udomchalothorn, T., Tschoep, H., et al. (2010). Arabidopsis has a cytosolic fumarase required for the massive allocation of photosynthate into fumaric acid and for rapid plant growth on high nitrogen. The Plant Journal, 62(5), 785-795. doi:10.1111/j.1365-313X.2010.04189.x
Sakai, K., Kinoshita, H., Shimizu, T., & Nihira, T. (2008). Construction of a Citrinin Gene Cluster Expression System in Heterologous Aspergillus oryzae. Journal of bioscience and bioengineering, 106(5), pp.466-472.
Timberlake, W. E., Boylan, M. T., Cooley, M. B., Mirabito, P. M., O'Hara, E. B., & Willett, C. E. (1985). Rapid identification of mutation-complementing restriction fragments from Aspergillus nidulans cosmids. Experimental Mycology, 9(4), 351-355. doi:10.1016/0147-5975(85)90008-8
Xu, G., Liu, L., & Chen, J. (2012). Reconstruction of cytosolic fumaric acid biosynthetic pathways in Saccharomyces cerevisiae. Microbial cell factories 11, p.24.
Yelton, M. M., Timberlake, W. E., & van den Hondel, C. A. M. J. J. (1985). A cosmid for selecting genes by complementation in Aspergillus nidulans: Selection of the developmentally regulated yA locus. Proceedings of the National Academy of Sciences of the United States of America, 82(3), pp.834-838.
Yelton, M., & Hamer, J. (1984). Transformation of Aspergillus nidulans by using a trpC plasmid. Proceedings of the National Academy of Sciences of the United States of America, 81(5), pp.1470-1474.

## Claims

1. A method for producing a vector for introducing a plurality of genes into a host cell, the method comprising the steps of:
a) providing for each gene of the plurality of genes, a DNA fragment comprising an expression cassette for expression of the gene in the host cell, wherein the DNA fragment has staggered 5' and 3' ends that are part of a restriction enzyme recognition sequence, which recognition sequence is not present elsewhere in the DNA fragment and which recognition sequence at the 5' end of the DNA fragment differs from the recognition sequence at the 3' end of the DNA fragment, and
wherein the overhang of the restriction enzyme recognition sequence at the 5' end is complementary with the overhang of the restriction enzyme recognition sequence at the 3' end; and,
b) combining the DNA fragments as obtained in a) by insertion of the DNA fragments into a unique restriction enzyme recognition site in a suitable vector, wherein the unique restriction enzyme recognition site in the vector has the same sequence as one of the two restriction recognition sequences at the 5' and 3' ends of the fragments in a), and wherein combination of a DNA fragment with a further DNA fragment produces a dysfunctional restriction enzyme site at the fusion site of the fragments.

2. A method according to claim 1, wherein the restriction enzyme recognition sequence at the 5' end of the DNA fragment and the restriction enzyme recognition sequence at the 3' end of the DNA fragment are recognition sequences of a restriction enzyme pair that provides complementary overhangs selected from the restriction enzyme pairs listed in Table 1.

3. A method according to claim 1 or 2, wherein for at least two genes of the plurality of genes the DNA fragments comprising the expression cassettes are assembled from subfragments comprising types of elements that together form a functional expression cassette, wherein the subfragments at least comprises in a 5' to 3' order:
i) a subfragment comprising an upstream regulatory type of element;
ii) a subfragment comprising a coding sequence type of element; and,
iii) optionally, a subfragment comprising a downstream regulatory type of element;
wherein the subfragments have staggered 5' and 3' ends, the overhang at the 5' end of each subfragment differs from the overhang at its 3' end, and the overhang at the 3' end of the subfragments comprising one type of element is complementary with the overhang at the 5' end of the subfragments comprising another type of element to allow assembly and operable linkage of the subfragments in a 5' to 3' order to form the functional expression cassette, preferably, wherein for all genes of the plurality of genes the DNA fragments comprising the expression cassettes are assembled from subfragments comprising types of elements that together form a functional expression cassette.

4. A method according to claim 3, wherein the staggered 5' and 3' ends are part of recognition sequences for restriction enzymes, which recognition sequences are not present elsewhere in the subfragment, preferably which recognition sequences also are not present in the DNA fragment comprising the expression cassette and/or in the vector.

5. A method according to claim 3 or 4, wherein the upstream regulatory type of element comprises at least a promoter and optionally further transcriptional and translational regulatory sequences, and wherein the downstream regulatory type of element comprises at least one of a transcription termination sequence and a polyadenylation signal and optionally further regulatory sequences.

6. A method according to any one of claims 3 - 5, wherein at least one of the subfragments comprising an upstream regulatory type of element and the subfragments comprising a downstream regulatory type of element are provided as an *E. coli* library.

7. A method according to any one of the preceding claims, wherein the vector is a cosmid vector, preferably wherein the vector is a binary vector system comprising a plurality of plasmid vectors and a cosmid vector, each plasmid vector comprising a plurality of 2 to 5 genes, each gene being a DNA fragment comprising an expression cassette, preferably wherein the plurality of genes present in the cosmid vector is assembled from each of the plurality of genes present in each of the plurality of plasmid vectors.

8. A method according to any one of the preceding claims, wherein the plurality of genes constitutes at least part of a metabolic pathway, wherein, preferably, the plurality of genes comprise additional gene copies of at least part of the metabolic pathway, modified genes of at least part of the metabolic pathway and/or genes of at least part of a metabolic pathway that does not natively occur in the host cell.

9. A method for introducing a plurality of genes into a host cell in a single step, the method comprising the step of transforming the host cell with a vector obtained or obtainable by a method according any one of claims 1 - 8, and, optionally, isolating a host cell comprising the plurality of genes.

10. A method according to claim 9, wherein the host cell is a microbial host cell, preferably a eukaryotic microbial host cell, more preferably a fungal host cell, most preferably a filamentous fungal host cell.

11. A vector obtained or obtainable by a method according any one of claims 1 - 8.

12. A vector of claim 11, wherein the vector is a binary vector system comprising a plurality of plasmid vectors and a cosmid vector, obtained or obtainable by a method according to claim 7.

13. A host cell obtained or obtainable by a method according to claim 9 or 10.

14. A method for producing a compound of interest, the method comprising the step of culturing the host cell of claim 13 under conditions conducive to production of the compound of interest, and, optionally, recovering the compound of interest.

15. A method for producing a compound of interest, the method comprising the steps of:
a) introducing a plurality of genes into the host cell in accordance with the method of claim 9 or 10, whereby the plurality of genes constitute at least part of a metabolic pathway involved in the synthesis of the compound of interest;
b) culturing the host cell obtained in a) under conditions conducive to production of the compound of interest; and,
c) optionally, recovering the compound of interest.


SEQUENCE LISTING

<110>  Wageningen Universiteit

<120>  Method for the single step introduction of a plurality of genes
       in microbial cells

<130>  P6046686EP

<160>  41

<170>  PatentIn version 3.3

<210>  1
<211>  499
<212>  DNA
<213>  Artificial

<220>
<223>  artificial sequence

<400>  1
tctagacgaa tgaggaggtg ttgcagaagt ggcgggctgt gacgaagggg gtaatttcgg      60

aagagaggca gaagaagatc gaggagattg tgttgaattt ggaagaggtg gaggatgtgg     120

ctggtgtttt gggcgagttg ttgagggaag agacggtgaa tgtgctgcag tagacggtta     180

ccccatttgg acggggatgg cttcatattt cccaagcgat gtcacgccat agaaagggca     240

catttacccg gtgcctgagc gaaactctac ttcgaagaca atgccaatgt ttaactatct     300

tgttttaatt gctaaatgca aacattccag gttcttccta atgccggcta aatcattcag     360

gctaaaccca agcgatgaag tcaatcggtc attctccggc gcatctaagc atctccgcaa     420

accgctataa aatctacccc agattcagtc cccggccacc tttctatccc cccccccaca     480

gactggctca accatgcat                                                  499

<210>  2
<211>  856
<212>  DNA
<213>  Artificial

<220>
<223>  artificial sequence

<400>  2
gcggccgcta gaaaatacta ttctgttgat ggctctaggg gatgagagtc agcctattac      60

tggatatcca tagtggtgat acgatgtata tagctctatg aagtaattag ttcaagtggg     120

aatacccctt tcacacatat agtatgctgt tattccgaaa tagggatcat ttctgattaa     180

tagtagcggt agcgatggtc acacacgact taatgttccc cattgtaccg gaagtaacaa     240

ttccagtgac ctcttagaag aaagacagca agaaaaagta agaaagggaa attgatcaaa     300

aaataaggcc atctacagcc tattcacatt tagccggatc tgcaatacag ctacagaaat     360

aaagtttgtt aggctgcttg ctagcatagc tcctactata ctaaaccaac acaatgggac     420

aataccccaa ttaaccagcc ctcactcaac acaagtgaat cctaccgaca acatccataa 480

accactgctt ccccacccag cacccttctt cacgatcaga tcacggagaa ttaccaacta 540

ctcttcgcat aaaacgtaaa caacggcctc gggccagcat ccgtccgact caaaagcaac 600

aaatccctcg ttcgcatact agccacatga acctgttgct ccgagacctc ctcaactggg 660

tcttcaaatg cccagaagac gctttcttct cgatatccat cggatactcg ctggccgctt 720

agacatatga acgatgagtc tcgtctgcca aaggaaacaa ccgtgttccc gaatccagtg 780

tcaaagtcgt aggtctggaa tttgaaaagt gttcgggcgt ttccttggag ggtcgggagt 840

gcgactgcag ggatcc 856

<210> 3
<211> 38
<212> DNA
<213> Artificial

<220>
<223> artificial sequence

<400> 3
agcttgcatg cctgcagatc tactctagac gaatgagg 38

<210> 4
<211> 38
<212> DNA
<213> Artificial

<220>
<223> artificial sequence

<400> 4
cctcattcgt ctagagtaga tctgcaggca tgcaagct 38

<210> 5
<211> 35
<212> DNA
<213> Artificial

<220>
<223> artificial sequence

<400> 5
gagacgaaag ggcctcgaga tacgcctatt tttat 35

<210> 6
<211> 35
<212> DNA
<213> Artificial

<220>
<223> artificial sequence

<400> 6

ataaaaatag gcgtatctcg aggccctttc gtctc 35

<210> 7
<211> 917
<212> DNA
<213> Artificial

<220>
<223> artificial sequence

<400> 7
   atgcatgaat ctaaggtgca aacgaatgtc cctcttccta aagctccact tacacagaag 60
   gcccgtggaa agcgcaccaa gggcatccct gcgctggttg caggcgcttg tgctggagct 120
   gtcgagattt ctatcacata cccctttgag agcgctaaga cccgcgctca gttgaagcgg 180
   cgcaaccacg acgtcgcagc catcaagccg ggtatccggg gttggtatgc gggttacggt 240
   gctacgctcg tgggtaccac gttgaaggct tcggtccaat tcgcgtcgtt caacatttac 300
   cgctccgctc tttccggccc caacggcgag ctctctacgg gagcatcggt cctcgcgggc 360
   ttcggcgctg gcgtcaccga ggccgtcctt gcggtgaccc ccgctgaggc catcaaaaca 420
   aagatcatcg acgcgcgtaa ggttggcaac gccgaactca gtaccacctt cggtgctatc 480
   gctggcatct tgcgtgatcg cggcccactc ggattcttct ccgcagttgg cccgaccatc 540
   cttcgtcaga gctccaacgc tgccgtgaag ttcaccgtct ataacgaatt gattggcctc 600
   gcccgcaagt acagtaagaa cggagaggat gtccacccct tggcctccac gctggtcggc 660
   agcgtgactg gcgtctgttg cgcctggtcc acgcagcctc tcgatgttat taagacccgc 720
   atgcaatccc tgcaggcccg ccagctctac ggcaacacgt ttaattgcgt taagaccctg 780
   ctccgcaacg aaggcatcgg tgttttctgg tccggagtct ggttccgtac aggtcgcctt 840
   tctctgacta gcgctatcat gttccccgtc tacgagaagg tctacaagtt cttgacccaa 900
   cctaactgag cggccgc 917

<210> 8
<211> 2357
<212> DNA
<213> Artificial

<220>
<223> artificial sequence

<400> 8
ctcgagctaa catacattcc gaaccgtgca gcccaaggcc gagcagttca actgcgctca 60

gcgcgctcat gccaacttcc ttgagaactc cagccaaact atgctcttcc tcctggtagc 120

tggactgaag tacccccagt tggcgactgg cctcggaagc atctgggtcc tcggtcgctc 180

actgttcctt tacggatatg tgtactccgg caagccgcgg ggtcgcggtc gtttgtacgg 240

cagcttctac ttgcttgcac agggagctct ctggggcttg acgtcttttg gagttgcgag 300

ggagttgatt tcctacttct aagtttggac tgaatccgtg gtgtgattga ggtgattggc 360
gatgtttggc tataccagct atatgtaata atctctactg tatactacta ttcaacgcat 420
tttactatgc gtgctgctag ggtcggcaat gacaatggca atctgactga cgtggtctat 480
ttctccatgt gcagcaggga atacgagctc caatggacct cgggagtggc acagtcaatg 540
gcaaggaaac tccgcctttg caggtgtggc tgaaccccac gggtcggagg cggagcaatc 600
cacccccgat gtggctggtg cgtggagggg ctcgcgatga ttttactgag cttgcttttc 660
ttgtcgacat tgaacattgt ccttggtctt ccttcagatt taagggtcag tcactgctac 720
atttctcagt agtatccgcg cacgtctctg gatttacgaa tcagggtcca ccagtcgaaa 780
cttcgaacta ctctcattat acaatcctct ttccattccc gcattaaccc ctccatcaac 840
accatgtcct ccaagtcgca attgacctac actgcccgtg ccagcaagca tcccaatgct 900
ctggcgaaga ggctgttcga gattgccgag gccaagaaga ccaatgtgac tgtctcggct 960
gacgttacca ccactaagga gctactagac cttgctgacc gtaggccgac ccgctactct 1020
gcctgattat gctgcatgca aacttattaa cggtgatacc ggactgcagg tctcggtccc 1080
tacattgccg tgatcaaaac ccacatcgat atcctctctg atttcagcaa cgagaccatt 1140
gagggactta aggctctcgc gcagaagcac aactttctca tcttcgagga ccgcaagttc 1200
attgacatcg gcaacacggt ccagaagcaa taccacggcg gtaccctccg tatctcggaa 1260
tgggcccaca tcatcaactg cagcattctc cctggtgagg gtatcgtcga ggctctcgct 1320
cagacggcgt ctgcaccgga cttcgcctac ggccccgaac gcggtctgtt gatcttggca 1380
gagatgacct ctaagggctc cttggctacc ggccagtaca ctacttcctc ggtcgattat 1440
gcccggaaat acaagaactt cgttatggga ttcgtgtcga cgcgcgcgtt gggtgaggtg 1500
cagtcggaag tcagctctcc ttcggatgag gaggactttg tggtcttcac gactggtgtg 1560
aacatttctt ccaagggaga taagcttggt cagcagtacc agacgcccgg atcggctatc 1620
ggccggggtg ctgacttcat tatcgcgggt cgcggtatct acgccgcgcc ggacccggtg 1680
caggctgcgc aacagtatca gaaggagggg tgggaagcct acctggcccg tgtcggcgga 1740
aactaatact ataaaaggag gatcgaagtt ctgatggtta tgaatgatat agaaatgcaa 1800
cttgccgcaa cggatacgga agcggaaacg gaccaatgtc gagcacgggt agtcagactg 1860
cggcatcgga tgtccaaacg gtattgatcc tgcaggctac tatggtgtgg cacaaggatc 1920
aatgcggtac gacgatttga tgcagataag caggctgcga agtagtaact cttgcgtaga 1980
gaaaatggcg acgggtggct gataagggcg gtgataagct taattgtcat cgcagataag 2040
cactgctgtc ttgcatccaa gtcagcgtca gcagaaatac gggacttccg aaagtatatg 2100
gcaaaattaa agaacttgac tctccagcaa tgttttgccc tgaccgtcgc taaaacgtta 2160

ctacccctat acccgtctgt ttgtcccagc ccgaggcatt aggtctgact gacagcacgg 2220

cgccatgcgg gcttgggacg ccatgtccgt cgcgtgataa gggttgatcc atgcagctac 2280

tatccttcca tcgttccatt cccatccttg tcctatctcc atccttgaaa ctttactagt 2340

ttagttggat gctcgag 2357

<210> 9
<211> 1475
<212> DNA
<213> Artificial

<220>
<223> artificial sequence

<400> 9
atgcatggtc gtggtgatac tgaaagtcct aatcctgcta ctacatctga aggttccggt 60

caaaatgagc cggagaagaa gggccgtgac atccctctct ggcggaagtg tgtcatcacg 120

tttgttgtgt cctggatgac tctggtcgtg acttttagct ctacctgtct gcttccggcc 180

gcccccgaga tcgctaacga gtttgatatg accgtggaga ctatcaacat tagcaacgcc 240

ggtgtccttg tggctatggg ctactcctcc ttgatctggg gccctatgaa caagctggtg 300

ggtcgtcgca ccagttacaa cttggccatc tcgatgctgt gcgcgtgctc cgctggtact 360

gccgccgcaa tcaacgagaa aatgtttatc gccttccgcg ttctctccgg cttgactgga 420

acgagcttta tggtttctgg ccaaaccgtc ctggctgata ttttcgaacc tgtctatcgc 480

ggtactgcag tgggcttctt catggcgggc actctctctg gccctgcgat tgcgtgcgtc 540

ggaggcgtca tcgtcacctt cactagctgg cgtgtgatct tttggctgca attgggcatg 600

agcggcctgg gtttggtcct ttccctcctt ttctttccga agatcgaagg cacgagtgag 660

aaggtttcta ctgcttttaa acccactact ctcgtctcga tcatctctaa gttcagtccc 720

acggatgtcc tcaagcagtg ggtgtatcct aacgtcttcc tggccgtttc cgcctgggaa 780

atctgcccct tgcacctgct cgagacgaag tgcagttgtc gcaagcagaa ggatctctgc 840

tgtggcttgc tggccatcac ccagtatagc attcttacct ccgcccgtgc catcttcaac 900

agccgcttcc acctcacaac tgcgctggtc agcggtctgt tctacctcgc gcctggcgct 960

ggtttcttga tcggctctct ggttggcggc aagctgtctg accggaccgt gcgtcgctat 1020

attgtcaagc gcggtttccg tctgccgcag gaccggttgc attctggctt gatcaccctc 1080

tttgccgtcc tcccagccgg aactctgatt tatggctgga ccctccagga ggacaagggt 1140

ggcatggtgg ttccgatcat cgccgctttc ttcgctggat ggggattgat gggctccttt 1200

aactgcctga acacatacgt ggccgtcgag gccctccctc ggaatcgcag cgctgtcatc 1260

gccggtaaat acatgattca atactcgttc tcggccggtt cttccgcctt ggttgtcccc 1320

gtcatcgatg ctctgggtgt gggttggact ttcacccttt gcgtcgtcgc ttccactatc 1380

gccggtctta tcaccgccgc catcgcccgt tggggtatca acatgcaacg ttgggccgag 1440

cgtgctttta acctgccgac acagtaagcg gccgc 1475

<210> 10
<211> 1484
<212> DNA
<213> Artificial

<220>
<223> artificial sequence

<400> 10
atgcataaac agagtgcgga cagcaacgct aagagcggag tcacggccga gatttgccac 60

tgggcgtcta atttagcaac agacgacata cccagcgatg tgctggaaag agctaagtac 120

ctgatcctcg atggtatcgc ctgtgcctgg gttggtgccc gtgttccttg gtccgagaag 180

tatgttcagg ctaccatgtc gtttgaaccc ccgggtgcct gtcgcgtcat tggatatggc 240

cagaagctgg ggccggtggc agctgcgatg actaactctg cttttattca ggcgactgaa 300

ttggatgact accactcaga ggcccccctt cactctgcgt cgatcgtgct gccggcagtg 360

ttcgcagcca gcgaggtcct agcagagcaa gggaagacga tttccggtat tgacgtcatt 420

ctggcagcca tcgttggctt tgagagtggt ccacgcatcg gcaaggcgat ctacggtcgg 480

gacctcctta acaatgggtg gcattgtggt gcagtgtatg gggctcccgc aggagctctg 540

gcgaccggca aattgcttgg actgactccg gactccatgg aagatgctct tggtatagct 600

tgcacccagg cttgcggact catgtcagcc caatatggcg gaatggttaa aagggtgcaa 660

catggattcg ccgctcgtaa cggcctctta ggcggcctgt tggcatatgg cgggtatgaa 720

gcgatgaagg gtgtgctcga acgttcatac ggaggttttc tgaagatgtt caccaagggc 780

aatgggcgtg agccccccta caaagaagaa gaagtggtgg ccggcttggg ctccttctgg 840

cacaccttca caatccggat taaactgtac gcttgctgtg ggttggtcca tggtcctgtc 900

gaggcgatcg aaaaactcca acggcgctat cctgagcttc tcaaccgggc caatctatcg 960

aatatcagac atgtatacgt gcagctaagc acggctagta acagccattg cggatggata 1020

cccgaggaaa ggccgatttc ttctatagca ggccaaatgt ccgtcgccta catccttgcc 1080

gtccagctcg ttgatcagca atgcctgtta gcccagttct ctgagtttga tgataatctc 1140

gaacgacctg aggtctggga ccttgcgagg aaagttactc cgtcgcactc cgaagaattc 1200

gatcaggatg gaaattgtct ttcagccgga cgcgtccgaa tcgagttcaa cgacggctca 1260

agtgtaaccg aaacagtgga gaaacctctg ggcgtcaagg aaccaatgcc aaacgaacgg 1320

attctacaca agtaccgaac gttggctggt tcggtcaccg atgagtcccg cgtaaaggaa 1380

atcgaagacc tcgtcctgtc cttggaccgg ttgacagata tcactcctct tctcgaactg 1440

ctcaactgcc cagtaaagtc gccactagtg taataggcgg ccgc 1484

<210> 11
<211> 401
<212> DNA
<213> Artificial

<220>
<223> artificial sequence

<400> 11
atgcatcagc cggtgcaacg tgccgccgca cgctcgttct tgcagaaata catcaacaag 60

gaaaccttga agtacatttt cacaacacac ttctggggac ccgtatcaaa tttcggtatc 120

cctattgcag ctatctacga tctgaagaag gaccctacgc taatctctgg cccaatgact 180

tttgctttag ttacctatag tggtgtgttc atgaagtatg cactttccgt cagccccaaa 240

aactacctgc tgtttggatg ccaccttata aatgagactg cccagctcgc ccaaggctat 300

cgctttctca agtacacgta tttcaccacc gacgaggaga agaaggctct cgataaggaa 360

tggaaagaga aagaaaagac tgggaaacag tgagcggccg c 401

<210> 12
<211> 398
<212> DNA
<213> Artificial

<220>
<223> artificial sequence

<400> 12
atgcatacat catccgtacg ttttgcattt cgacggttct ggcaaagtga gaccgggccg 60

aagacggtgc acttctgggc tcctactttg aaatggggtc tggttttcgc tgggttctca 120

gatatgaaaa gaccggtcga aaaaatttct ggtgctcaga atctctcgct gctatctacc 180

gccctgattt ggacacgttg gtccttcgtc atcaagccac gcaacatctt actcgcatct 240

gtcaactcgt ttctttgtct taccgcgggc tatcagttag gaaggatcgc caactaccgc 300

atacggaatg gagattccat ctcgcaattg tgcagctaca ttctcagcgg agccgacgaa 360

agcaagaagg agatcactac gggccgctga gcggccgc 398

<210> 13
<211> 449
<212> DNA
<213> Artificial

<220>
<223> artificial sequence

<400> 13
atgcatgcaa gcgcttttaa ttttgccttt cgccgatttt ggaatagtga aacaggccct 60

aaaacagtgc acttctgggc cccaaccttg aaatgggggc tggttttcgc aggtctaaat 120

gatattaagc gtccagtgga gaaggtatca ggagcccaaa acttgtctct tttagcgacg     180

gcactgattt ggacgcgttg gtcgttcgtc atcaagccca agaactatct cctcgcttcc     240

gtcaacttct tcctgggatg caccgccggc taccatctca ctagaatcgc taacttccgc     300

atacggaacg gtgactcttt caaacaggtt atccactaca ttatcaaagg ggagactcca     360

gctgccgtcg ctgcgaagca gaccgcatcc acctcgatga acaagggtgt gatcggcact     420

aatccgccca ttacccacta ggcggccgc                                       449

<210>  14
<211>  837
<212>  DNA
<213>  Artificial

<220>
<223>  artificial sequence

<400>  14
atgcatgctg caattaaagc aatcaatgcg aagatacggt ctaacaaggc gctggactat      60

ttctgtagca cccgtacgtt caatcttcac cgttaccctc cccgtgttcc tgaccgagat     120

cggccgacaa gaatgcctgc cgtaagacat gttctgaagg ggttgttgag caacttcagt     180

gaatcctttc tgcacatgtt atcggtcacg atcacacagg tctttcctta gtcaactctg     240

attaacaaaa agcaatttct cttggctaac aatgatctca actagacttc tggggccctg     300

ccagtaattt tggtatcccg attgcagcgg tcatggatac tcagaaggac ccagaaatgt     360

acgtcgctac aattacctct ccccccctcc gcggagttct ttctcttgaa cataagtgca     420

catcaaatca agctttgaga gcttatccag atagcccaaa cgaacatacc agcaccttcc     480

taaccaatca tacgattgct aactgtcatt ttatctacac agtatctccg ggcaaatgac     540

cggtgctctc gttatctact cggggacgtt catgagatac gcccttgcag tgtcgcccaa     600

aaactatttg ctatttgcgt gccacgccgt caatttctca gcacagctta cacaaggtta     660

ccgctacctg aactattgga agtacgtcac ggaatggctc tttcttccca ccctcattga     720

gaaacacaag gataatgctg actttcgcat caacagctgg ggaggacgtg aagctcagct     780

cgccgaggct gccaagcagg gcaaggatgc cacggaggct ggcgcttagg cggccgc        837

<210>  15
<211>  797
<212>  DNA
<213>  Artificial

<220>
<223>  artificial sequence

<400>  15
atgcatagtt ccagggttgg tctccgtttc tttcaaaaca cgagagcagc tttccgaaat      60

gcgtatggac cattccggcg gcctggggcc caaggcttca gattccagtc aagtgatgct 120

gcggcagcag agcaacagag cactttccag cgtctatgga acagcccggt cggggtgaag 180

acggtgcatt tttggtgagt ccagactcgg atattatgct attgcatttg cttttttctt 240

ctatcatgtg aacgtttgcg ttgtactgta ttttaacatt gatgtgtcat agggcccccg 300

ttatgaaggt acgaaaccat gcaccgtgct gaaacccgtt cctcgatcca acttctttga 360

ttcgcgataa tcgtcctact atatctttgt gctgttcgac tgactcaatc agtgggctct 420

tgtcattgcc ggaatatccg acttttctcg ccctgcggag aagctgtccc ttacacaaaa 480

tggcgccttg atggcaactg gttgcatctg gacccgctgg tgcatgatta tcacccccaa 540

gaattacctg tacgcacacc aaccccgatc tatgaatatt gtgggaaaga atactgactg 600

gctaggttag ctgctgtcaa cttctttctc ggatgtgtgg gtgtcgtcca ggttggccga 660

atcatcaact accgcaggtc agttgacggc tcgaccaccg cggccgtgaa agacctggaa 720

caggaagtag cggacgacgc caaggccgtg gctacacagg cgaaagctgc agtcgagaaa 780

tctacctagg cggccgc 797

<210> 16
<211> 393
<212> DNA
<213> Saccharomyces cerevisiae

<400> 16
atgtctcaac cggttcaacg cgctgcagca cgctcattcc ttcaaaaata catcaataaa 60

gaaactttga aatatatttt cacaacacac ttctggggtc ccgtatcaaa tttcggtatc 120

ccaattgctg ctatatatga tctgaaaaaa gaccctacac taatctctgg cccaatgact 180

tttgctttag ttacctattc aggtgttttc atgaagtatg ctctttcagt atcacccaaa 240

aactacttac tgtttggatg ccaccttatt aatgaaactg cgcaattagc tcaaggctat 300

aggtttctca aatacacgta tttcacaaca gatgaggaga agaaagctct agataaggaa 360

tggaaagaga aagaaaaaac tggtaaacag taa 393

<210> 17
<211> 390
<212> DNA
<213> Saccharomyces cerevisiae

<400> 17
atgtctacat catccgtacg ttttgcattt aggcggttct ggcaaagtga gacaggcccc 60

aagacggtgc atttctgggc tcctactttg aaatggggtc tggttttcgc tggattcagc 120

gatatgaaga gaccggtgga aaaaatttct ggtgctcaaa atttgtcgct gctatctact 180

gcgctgattt ggactcgttg gtcctttgtc atcaagccaa gaaacatctt gttggcttct 240

gtcaactcgt ttctttgtct gaccgctggc tatcaattgg gtagaattgc caactacagg 300

atacggaatg gcgactctat atcgcaattg tgtagctata ttctcagcgg cgccgacgaa 360

agcaaaaagg aaattactac gggcagataa 390

<210> 18
<211> 381
<212> DNA
<213> Saccharomyces cerevisiae

<400> 18
aaaacagtac acttctgggc cccaactttg aagtgggggc tggtcttcgc agggctaaat 60

gatattaaga ggcctgttga gaaggtatca ggagcacaaa atttatcttt attagcgacg 120

gcactgattt ggacgcgttg gtcgtttgtc atcaagccca agaactatct gttagcttcc 180

gtcaattttt tcctgggttg cactgcaggc taccatctaa caagaattgc taactttagg 240

atacggaacg gtgattcttt taaacaggtt attcactaca taataaaagg ggagactcct 300

gcagccgtcg cagcaaagca aactgcatcc acatcgatga acaaaggtgt gatcggtact 360

aatccgccaa taacgcactg a 381

<210> 19
<211> 1022
<212> DNA
<213> Artificial

<220>
<223> artificial sequence

<400> 19
atgcatgcgg cctctcgggt gttctctatt gcagcaaagc gttctttctc aacgagtgct 60

gcgaatcttt ccaaagttgc cgttctgggc gcggctggcg gaattggcca gcccttgagt 120

ttgctgctaa aggagaatcc acatgtcacc cacctttcac tttacgatat tgtgaacact 180

ccgggagtcg ctgcagacct aagccatata aataccaaca gcaaggtcac tggccacacc 240

cccgagaatg atggtctcaa gacagcactg gaaggggctc acgttgtggt tataccggct 300

ggcgtgccta ggaaacctgg catgacgcga gatgatttat tcaacaccaa tgcgtccata 360

gtacgtgacc tggcagaggc tgctgccaaa cactgccccg acgcacattt ccttatcatc 420

tccaaccctg tcaactccac tgtgcccatc tttgccgaga ccttaaagaa ggctggtgta 480

ttcaacccta agagattgta tggcgtgacg acgcttgacg tggtccgggc ctcacgcttc 540

gtggccgagg tcaagaactt ggaccccaac gatgtcaaag tgaccgttgt cgggggtcat 600

tcgggcgtga caatcgtccc actcctctcg cagaccgggc tcgaattcag caaggaggaa 660

ctagatgcgt tgacgcaccg catccaattc ggaggtgacg aggtcgtaca ggccaaaaat 720

gggactggtt cggtcactct cagtatggcc tttgcgggcg ctcgattcgc caactccgtg 780

cttgaggcca cagtgggagg aaaaaagggt gttgttgaac cgtcctttgt gaagtctgat 840

gtgtttgcta aagatggggt tgaatacttc agcacaaata tcgagctggg accggaaggt 900

gtggagaaga tcaatgaact cggacaaatc tcggactatg agaaggaact gattgctaag 960

gccgtaccag aactgaagaa gaacattgcg aaaggaaaca gctttgttca gtaagcggcc 1020

gc 1022

<210> 20
<211> 1858
<212> DNA
<213> Artificial

<220>
<223> artificial sequence

<400> 20
atgcattcct ttgaagaaga agaccggaag tatttggatg atgtccaggc cgtccagaag 60

tggtggcagg actcaagatg gagacacacc aagcggccct ttactgctga gcaaattgtg 120

gctaagcgcg gcaccctgaa gatcgaatac ccttccaatg ctatgagtaa gaagctatgg 180

aagattttgg agagcaattt cgaggttcgc atgtccctgc tctgtggtgg ttgacatatt 240

tcctaaattt atctgtcctc cattagaaaa aagtacctag cttcacttat ggttgtctcg 300

aacccacaat gctcacccag atggccaaat accttgacac cgtctatgtc tctggctggc 360

aaagctcgtc caccgcctca tctaccgacg aaccttctcc ggaccttgcc gactacccaa 420

tggtaggtct caaaaggatg ttgttggttg tgggatcact gctaaaacat gagttagaat 480

acagtcccaa acaaggtcaa ccagttattc ctggctcagc tattccatga cagaaaacag 540

cgcgaagaaa ggatcacaac tcccaaggag cagcgtggca aagtggcaaa tgttgactat 600

ctccgcccaa taattgcgga tgccgatact ggacatggtg gcttgacggc cgtgatgaag 660

ctgaccaagc tttttattga gcgtggggct gctggtattc acattgaaga ccaagctccg 720

ggaaccaaga agtgcgggca catggccgga aaggttcttg ttcctatcag tgaacacata 780

aaccgtctgg tcgctatccg tgctcaagcg gatataatgg gtatgtagca tcctcattcg 840

cttaatatga atggcatcaa ctgactctat tcaggcaccg atctccttgc aatcgctcga 900

acggactccg aagctgccac tctcattacc tccacaattg attacaggga tcatgccttc 960

gtcgtgggat caacaaaccc caacctgcag cccctcaatg acctcatggt tgccgcagag 1020

caggctggca aaaacggaaa cgagctacaa gccatcgaag accagtgggt tgctcaagct 1080

ggcctgaagc ttttctcgga ggctgtcatc gataccatca ataagagctc ctctggtaac 1140

aaaaaagccc tcatcgatga gtacctgaag aaggccaagg ggaagtcgaa ccgcgaagca 1200

agggctattg ccaaagacat cactggcgta gacatctatt gggactggga tgcacctcgc 1260

acacgggagg ggttctaccg ataccaagga ggtacgcagt gtgccgtgaa ccgagccatc 1320

gcctatggcc catttgcaga cctcatttgg atggagtcga aattgccaga ctatgcccaa 1380

gcgaaggagt ttgcagacgg tgttcacgcc gtctggccgg agcagaagta agtgttgtcc 1440

tgatttgcat tgcatcagcg gagacttcga tactaacctg accctttagg ttagcgtata 1500

acttgtcgcc ctcattcaac tggaagaagg cgatgccgcg agatgagcaa gagacataca 1560

tacaacgcct gggagcactg ggatactgct ggcaattcat cacccttgcg ggtttgcaca 1620

cgactgcgct gatcacggat cagtttgcca aggcgtacgc gaagcagggc atgcgggcct 1680

acggtgaact cgtgcaagaa ccggagatgg agcagggggt ggatgtggtg actcatcaga 1740

aatggagtgg tgcaaattac gtagataata tgttgaaaat ggttacgggg ggcatcagca 1800

gtacggccgc aatggggaaa ggcgtcacgg aggatcagtt caagcattga gcggccgc 1858

<210> 21
<211> 974
<212> DNA
<213> Artificial

<220>
<223> artificial sequence

<400> 21
atgcatcaga aaaagaaggc gagccacccg gcgatcaatc tcatggctgg gggcactgca 60

ggtcttttcg aagcgctctg ttgccaccct ctggacacaa tcaaggtgcg aatgcaaata 120

taccgtcggg tagcgggtat cgagcacgtt aaacccccag gatttatcaa aactgggcgt 180

accatttacc aaaaggaagg gttcttggcg ctgtataagg gactaggcgc ggtggtcatc 240

ggcatcatcc ctaagatggc aatccgcttc tcgtcttacg agttctacag aaccttactt 300

gttaacaagg aatcaggaat tgtgtctacc ggtaatacgt ttgtcgctgg cgttggggct 360

ggaatcaccg aagctgtcct tgtcgtgaac ccgatggaag ttgtcaagat tcgtctccaa 420

gcccagcacc tgactcccag tgagccaaat gctgggccta aatataacaa cgccattcat 480

gcagcctaca ctattgtcaa ggaagaaggg gtttctgccc tctaccgggg cgtatccttg 540

actgcggccc gacaggcaac taatcaggga gctaacttca cagtttatag caaactgaag 600

gagtttctcc agaattacca tcagatggat gtgctgcctt cgtgggagac gtcctgcatc 660

gggctaattt cgggtgcgat tgggccgttc tccaacgctc cacttgacac gataaagacg 720

aggctgcaaa aggataagtc catcagcttg gagaaacagt ccggcatgaa gaaaatcata 780

acgattggtg cacagctcct gaaggaggag ggttttcgcg ccttatacaa gggtattacc 840

ccccgcgtca tgcgcgtggc tcccggtcag gccgtcacct tcaccgtgta tgaatatcgc 900

aagggagcat tccagaaaat ctgggtatac tcacggagaa tgacacacca aagccaaagt 960

cattaggcgg ccgc 974

<210>  22
<211>  25
<212>  DNA
<213>  Artificial

<220>
<223>  artificial sequence

<400>  22
ccatgcattc gacctcgttt aggga                                              25


<210>  23
<211>  27
<212>  DNA
<213>  Artificial

<220>
<223>  artificial sequence

<400>  23
atgcggccgc tcaatcggag ggaccaa                                            27


<210>  24
<211>  1409
<212>  DNA
<213>  Artificial

<220>
<223>  artificial sequence

<400>  24
atgcattcga cctcgtttag ggaggagagg gacaccttcg ggccgattca agttccttcc      60

gataaattgt ggggagccca gacgcagaga tcgctgcaga acttcgaaat cggtggtgag     120

cgcgagcgaa tgcctgaacc aattgtccgc gcttttggcg tcttgaagaa atgcgctgcc     180

aaggttaaca tggaatacgg tcttgatccg acgattggga aagcaattat gcaagctgct     240

caggaagtag ctgagggaaa gctcaatgat catttccccc ttgttgtctg gcaaactggt     300

agtgggactc agagtaatat gaatgctaat gaggtcattg ctaatagagc agctgagatt     360

cttggtcgca aacgtggtga gaaatgtgtg cacccaaatg accatgtgaa cagatcacaa     420

tcttctaacg acacttttcc taccgtcatg cacattgcag ctgcaaccga gattaattcg     480

aggctcatcc ctagtttaaa aactttgcat agcactttgg aatctaagtc cttcgagttc     540

aaagatattg tgaaaattgg aagaactcac actcaagatg ctacaccttt gacactagga     600

caagaatttg gtggctatgc tactcaagtt aagtatggac taaatagagt cacctgcact     660

cttcctcgcc tctatcagct tgcacaaggt ggaactgcgg ttgggacagg attaaacact     720

aagaaagggt ttgatgtaaa gatagctgct gcagtagctg aagaaacaaa cttgccattt     780

gtcactgcag aaaacaagtt tgaagctctg gctgcacacg atgcttgtgt tgaaaccagt     840

gggtcactta acacaatcgc cacatcgctg atgaagatcg ccaatgatat acgttttctt     900

ggaagtggcc caagatgtgg tcttggtgaa cttgtactac ctgaaaatga gccaggaagc 960

agtatcatgc ctgggaaggt aaatcctaca cagtgtgagg ccttgactat ggtttgtgca 1020

caggttatgg gaaaccatgt agctgtgaca gttggtgggt caaatggtca tttcgaattg 1080

aatgtattca agccggtgat cgcgagtgct cttttacatt ccgtcagatt aatagcagat 1140

gcttcagctt cgttcgagaa aaactgtgtg aggggcattg aggccaacag agaaagaatc 1200

tcaaagctat tgcacgagtc tcttatgctt gtgacatcat tgaatccgaa aatcgggtat 1260

gacaatgctg cagcagttgc caagaaagct cacaaagaag gatgtacatt gaaggaagcg 1320

gctctgaatt taggcgtgct tactgcggaa gagtttgata cacttgttgt tcctgagaag 1380

atgattggtc cctccgattg agcggccgc 1409

<210> 25
<211> 25
<212> DNA
<213> Artificial

<220>
<223> artificial sequence

<400> 25
ccatgcatga gaccgatgcc ttcgg 25

<210> 26
<211> 27
<212> DNA
<213> Artificial

<220>
<223> artificial sequence

<400> 26
atgcggccgc ttatttcttc tccttgg 27

<210> 27
<211> 1508
<212> DNA
<213> Artificial

<220>
<223> artificial sequence

<400> 27
atgcatgctt tgacaatgca gtttgaagga gagaagaaaa acgtatccga agttgcagac 60

gtaaccctca agcaagaaga tgaacaacaa gaacgtagat cttattcgac gccgtttagg 120

gaagagagag acacctttgg cccgatccaa gttccttccg ataaattatg gggagcacag 180

acgcagagat cgcttcagaa cttcgagatt ggtggtgacc gcgagcgaat gcccgaacca 240

atcgtccgag cttttggtgt cttgaagaaa tgtgctgcca aggttaacat ggagtatggt 300

cttgatccaa tgattgggga agccataatg gaagctgcac aagaagtagc agaaggaaag 360

ctcaatgatc atttccctct tgttgtatgg caaactggta gtgggacgca gagtaatatg 420

aatgctaatg aggtcattgc caatagagca gctgagattc ttggtcacaa acgtggtgaa 480

aaaattgtgc acccaaatga ccatgtgaac agatcacaat cttctaatga cacttttcca 540

actgtcatgc acattgcagc tgcaaccgag attacttcga ggctaatccc tagtttgaaa 600

aatttgcata gctctttgga atctaagtcc ttcgagttta aagatatagt gaaaatcgga 660

agaactcata ctcaagatgc tacacctttg acattaggac aagaatttgg tggctatgct 720

actcaagttg agtatggact taatagagtc gcatgtactc taccccgcat ctatcagctt 780

gcacaaggtg gaactgctgt tgggaccgga ttaaacacta agaaagggtt tgatgtaaag 840

atcgctgctg cagtagctga agaaacaaac ttgccattcg tcaccgcaga aaacaagttt 900

gaagctctgg ctgcacacga tgcttgtgtt gaaacaagtg gatctcttaa cacaatcgcc 960

acatcattga tgaagattgc caatgatata cgttttcttg gaagtggtcc aagatgtggt 1020

cttggtgaac tttctctgcc tgagaatgaa ccaggaagca gtattatgcc tggaaaggta 1080

aatcctacac agtgtgaggc cttgactatg gtttgtgctc aagttatggg aaaccatgta 1140

gccgtgacaa ttggtgggtc gaatggtcat tttgaattga atgtattcaa gccggttatc 1200

gcaagcgctc tcttacattc cattagacta atagcagatg cttcagcttc atttgagaaa 1260

aactgtgtta gaggcattga ggccaacaga gaaaggatct caaagctatt gcacgagtct 1320

cttatgcttg tgacatcatt gaatcctaaa attggctatg acaatgctgc agcagtagcc 1380

aaaagagctc acaaagaagg atgcacatta aagcacgcag ctatgaagtt aggtgttctt 1440

acttcggaag agtttgatac tcttgttgtt cccgagaaga tgattggtcc atctgattaa 1500

gcggccgc 1508

<210> 28
<211> 25
<212> DNA
<213> Artificial

<220>
<223> artificial sequence

<400> 28
ccatgcatga gaccgatgcc ttcgg 25

<210> 29
<211> 27
<212> DNA
<213> Artificial

<220>
<223> artificial sequence

<400> 29

atgcggccgc ttatttcttc tccttgg                                          27

<210>  30
<211>  1400
<212>  DNA
<213>  Artificial

<220>
<223>  artificial sequence

<400>  30
atgcatgaga ccgatgcctt cggtgagatc gaggtccccg ccgacaagta ctggggtgcc      60

cagacccagc gctccctggg caacttcgac atcaaccagc cccaggaccg catgcctgag     120

cccgttgtca aggctttcgg tatcctcaag ggtgctgctg ctgaagtgaa catgaagttc     180

ggccttgacc ccaagatcgg cgaggccatc aagcaggctg ccgccgaggt tgcggagggc     240

aagctgatgg accacttccc cctcgtcgtc tggcagaccg gttccggtac ccagtccaac     300

atgaactcga acgaggtcat ctccaaccgc gccattgaga tcctgggcgg cgagaagggc     360

tccaagaagc ccgtccaccc caacgaccac gtcaacatgt ccgcctcctc caatgactcc     420

ttcccgaccg ctatgcacat tgctgccgtt gtggagctgg agaacaccct cctgccttcc     480

ctgaggagcc tgcgcgatgc tctccaggtc aaggttgaga agttcgacaa gatcatcaag     540

atcggtcgta ctcacctgca ggacgccacc cctctcaccc tcggtcagga gttctccggc     600

tacgtcgctc agctcgaccg caacattgag cgtgtcgaga ctagcatccc ccacctccgc     660

tacctggctc agggtggtac cgccgtcggt actggtctga acaccttcaa gggcttcgac     720

gaggctatcg ctgctgaggt caccaagttg accggcaccg agttcaagac tgcccccaac     780

aagttcgagg ttctggccgc ccacgactcg attgtcgagg cttccggtgc cctgaacacc     840

ctggcctgct ctctgttcaa gattgcccag gacatccgtt accttggatc cggtccccgc     900

tgcggtcttg gtgaactggt cctccccgag aacgagcctg gctcttccat catgcccggc     960

aaggttaacc ccactcagtg cgagtccctt accatggtct gctcccaggt catgggtaac    1020

cacgtcgctg ccactgtcgg cggcatgaac ggtcagttcg agctcaacgt gttcaagccc    1080

ctcatgatcc gcaacctgct gcacagcgtg cgcatcctgg ccgatggcat ggccagcttc    1140

gagaagaacc tggtgcacgg tctggaggcc aacgagcccc gcatcaactc tctcctccac    1200

gagagtctga tgttggtcac ctgcctgaac cccgtcattg gctacgacat ggcctccaag    1260

gtcgccaaga acgcccacaa gaagggcctc actctgaagc agagtgctat ggagctgaag    1320

gctctgagcg aggaggactt tgacaagtac gtccgcccgg agctgatgct gagccccaag    1380

gagaagaaat aagcggccgc                                                1400

<210>  31
<211>  1448

<212>  DNA
<213>  Artificial

<220>
<223>  artificial sequence

<400>  31
atgcataact ctccacgcct gttctctagc gcttccgctg cactccaaaa gtttcgcgct      60

gagcgcgata ctttcggaga tcttcaggtc cctgccgacc gttactgggg tgcccagacc     120

cagcgctcgt tgcagaattt cgatattggt ggtcccaccg aacgtatgcc ggagcccctt     180

attcgcgctt tcggagtgtt gaaaaaggcc gcagccacag ttaacatgac ctacggtctg     240

gaccccaagg tgggcgaggc tatccaaaag gcggctgacg aagtcatcga tggatccctc     300

atcgaccact tccctctggt cgtctggcaa accggcagcg gcacgcagac aaagatgaat     360

gtcaacgagg tcatctctaa ccgtgcaatc gagcttctcg gcggtgagct gggatccaag     420

gcccctgtcc accctaacga ccacgtcaat atgtcgcagt cctctaacga caccttcccc     480

accgccatgc acgtggcggc agtggttgaa atccatggac gtctgatccc tgccctcacc     540

accctgcgcg acgccctgca ggctaagagt gcagagttcg agcacatcat taagattggt     600

cgcacccatc tgcaggacgc cactccgttg acccttggcc aggagttttc tggctacacc     660

caacagctca cgtacggtat cgctcgggtt caaggcacgc tcgaacgcct ttacaacctt     720

gcgcagggcg gtactgccgt cggcacaggc ttgaacaccc gtaagggatt tgatgctaag     780

gtggccgagg ccattgcttc cattaccggc ctccccttta agaccgctcc aaacaagttc     840

gaggcccttg cggctcatga cgccctggtg gaggcacatg gcgccttgaa cacggtcgcg     900

tgctcgctga tgaagattgc gaacgatatc cggtacctcg gctctggacc gcgctgcggt     960

ctgggtgagc tcagcttgcc ggagaacgag cccggtagtt ctatcatgcc aggcaaggtt    1020

aacccgaccc agtgtgaggc gatgactatg gtctgtgctc aggtcatggg aaacaacact    1080

gcgatcagtg ttgccggatc caacggtcag ttcgaattga acgttttcaa gcccgttatg    1140

atcaaaaacc tcattcagtc gatccggctg atttcggacg cctcgatcag cttcacgaaa    1200

aactgcgttg tgggtatcga agctaatgag aaaaagattt cgagcatcat gaatgaatcc    1260

ttgatgctcg tgactgccct caatccccac atcggttatg ataaggctgc taagtgcgcc    1320

aaaaaggccc acaaggaggg cactactctt aaggaggcag ccctgagcct gggctatctc    1380

acatccgagg agttcgatca atgggtgcgt cccgaagata tgatcagcgc caaggattaa    1440

gcggccgc                                                              1448

<210>  32
<211>  1583
<212>  DNA
<213>  Artificial

<220>
<223>  artificial sequence

<400>  32
atgcatgaaa ttacccactt ctctcaccca acgaccgaaa gcaaagtaaa ggctggttct      60

tttacagaaa acgtccagga tgagcccaat cggtcccaca tcccagaaca tctacgtatt     120

ccgattcccg aaaaatcaag cactcactac ccggccttca ttacccgtac gacgcaggcc     180

gtgagccatg tctgctgtac atacctgatc tctcccttca agaagatcct tcgcatacta     240

cagaaacgaa ctgggctcac cttctggatc atcctggcta tggtcatcgg catcttgatt     300

ggtagatttg ctcctgaggc aggtgtggct atcaagccac tgggggatgc gttcattcag     360

atgataaaaa tcatcattgt tccccttgtc ttcagtgtgc taatcatcgg gatcgctggc     420

catggggatg atattgccaa ggtgggcaag ctggccataa agaccataat ctatttcgag     480

gtagttacca ccctggccct tgccgttggt ctcatcatgg ccaaccttat caagccggga     540

catggtgtta tcttacccgt cggacaggac acgtcgtcga tcgaggccat ggctgagaca     600

gaaaagacgt ccatcacttg gagcggtgag atgttcatga tcattccaga aaatttcttc     660

gttgccgctg ttggaaacaa agttctagcg atagtgtttt gtgcagtcat gtttgcttgc     720

tcgatgatga aggcggataa gaagtcaaag aaggtcatgc tggagataaa tgaggctctt     780

tctcaggtta tgtttaagtt tgttgggttg attatgaact atgcacctat cggtatcgga     840

gctgccttgg cggctactgt aggcaagaat ggtatcagtg tccttgcgaa tctaggaaaa     900

ctgatcgggt gcgtctacgc atcattggtt atttcttgat ttgtgattct ggtaccgatt     960

atgctgatcg cacggattcc tgtcattggc ttcttcaagg cgataggaca gccttggtta    1020

ctcgcattca gttcggcctc tagtgagtcc gcactgcctt tggcattcga acgcatgcga    1080

gagtttggtt gcacgaacgc gttgactgga ttcgtgattc cctgtggcta ttcctttaac    1140

ctggacggga ccacactcta tctgtccttg gccaccattt tctctgctca agcagccggc    1200

atgaatttac cgatcagcac acaactctct atcatgggca ccctcatgct ttcctccaag    1260

ggagtggctg ccattcctcg cgcgagcctc gtcgtgctct caggcacact cagtcaatac    1320

aacatcccac tcgaagctgt cttgatgata atgggtgtgg acgcgatcat ggacatggct    1380

aggacttccc tcaacgtgct cggcaactgt ctcggctgct gcgtgatggc aaggatcgag    1440

ggatcatttc gaggagagga gtggcggatg gaggaggaag acagacgcta taaaaagtac    1500

ctggaggaac aagaaaaata caactcggcc gatgatctta gcaatgtgat tgtcatccgt    1560

gacgagaaac aataggcggc cgc                                            1583

<210>  33
<211>  5693
<212>  DNA
<213>  Artificial

<220>
<223>  artificial sequence

<400>  33
atcgatccgg gcaacgttgt tgccattgct gcaggggggg gggggggggg gggggggggt      60

tgacttccat tgttcattcc acggacaaaa acagagaaag gaaacgacag aggccaaaaa     120

gcctcgcttt cagcacctgt cgtttccttt cttttcagag ggtattttaa ataaaaacat     180

taagttatga cgaagaagaa cggaaacgcc ttaaaccgga aaattttcat aaatagcgaa     240

aacccgcgag gtcgccgccc gtaacctgtc ggatcaccgg aaaggacccg taaagtgata     300

atgattatca tctacatatc acaacgtgcg tggaggccat caaaccacgt caaataatca     360

attatgacgc aggtatcgta ttaattgatc tgcatcaact taacgtaaaa acaacttcag     420

acaatacaaa tcagcgacac tgaatacggg gcaacctcat gtcaaccccc cccccccccc     480

cccccccccc ctgcaggcgg agaactggta ggtatggaag atctctagag aattagcccg     540

cctaatgagc gggctttttt tgaattcgag ctcgcccggg gatcgatcct ctagagtcct     600

gatgcggtat tttctcctta cgcatctgtg cggtatttca caccgcatat ggtgcactct     660

cagtacaatc tgctctgatg ccgcatagtt aagccagtat atacactccg ctatcgctac     720

gtgactgggt catggctgcg ccccgacacc cgccaacacc cgctgacgcg ccctgacggg     780

cttgtctgct cccggcatcc gcttacagac aagctgtgac cgtctccggg agctgcatgt     840

gtcagaggtt ttcaccgtca tcaccgaaac gcgcgaggcc cagctggctt atcgaaatta     900

atacgactca ctatagggag accggaagct taggatccta tgtattctat agtgtcacct     960

aaatcgtatg tgtatgatac ataaggttat gtattaattg tagccgcgtt ctaacgacaa    1020

tatgtacaag cctaattgtg tagcatctgg cttactgaag cagaccctat catctctctc    1080

gtaaactgcc gtcagagtcg gtttggttgg acgaaccttc tgagtttctg gtaacgccgt    1140

cccgcacccg gaaatggtca gcgaaccaat cagcagggtc atcgctagaa atcatcctta    1200

gcgaaagcta aggatttttt ttatctgaat tctagccaga tccccgggcg agctgcggcc    1260

tgatttatgc tggttactgt tgcgcctgtt agcgcggcaa cgtccggcgc acagaagcta    1320

ttatgcgtcc ccaggtaatg aataattgcc tctttgcccg tcatacactt gctcctttca    1380

gtccgaactt agctttgatt tctgcgatct tcgccagagc ctgtgcacga tttagaggtc    1440

taccgcccat gacaggaagt tgttttactg gttcagggat cgcctcacca cggttaattc    1500

tcgcagtcat atggacaagc tcatctgcgg ccttacggcg taattccgca tcagtaagcg    1560

cattggcccg catgttctga tacaggttgg taaccagcca gtagtgcgcg tttgatttcc    1620

acggataaga ctccgcatcc ggatacaggc ctcgcttccg gcaatactcg taaaccatat    1680

caaccagctc gctgacgttt ggcagtccgg cggtaacgga tgcttcttcc cggcaccatg    1740

caacaaactg cccgggtgat ggcagaaatg gtcgattctg ccgacgggct acgcgcattc 1800

ctgcgttaac ctgttccatc gtggtgatcc cgttttcccg aaaagccaga acccactggc 1860

gacggatttc gttcacttcg ttctggtcac ggttagccag gctcgccggg aaagttgcca 1920

gtaactggct gaacacaccg ttgatgatct gcgctacctg ctgtacctgc ggcttttcgt 1980

cgtactgttc cggcatgttg ttggcgatcc gacgcatctg ctcacggtca aagttaacca 2040

tctgtgcggc gatgtttttc atagatccac cccgtaaatc cagtctgtgt ttgtcaggtc 2100

gagttttggt ttgctggctg tcacgcctgc ctgttgcttg ttacggttga tttcgagttg 2160

ggtccactta tcgcggagtt tggccgggct cagcacgtta ccggaccaga agttgtcctg 2220

gcatgcccag cggaacagca cacacatgtc gcggtggtta cgtccgtcac gttcacgcat 2280

caggcggata tcgttagccc acccagcaaa attcggtttt ctggctgatg gtgcgatagt 2340

cttcaccatg tcaaacatcc actctgcggc ggtcaggtct tctgctgtcc cccacttgct 2400

gccgctctga attgcagcat ccggtttcac cacagaaagg tcgttttctg gctggtcaga 2460

ggattcgcca gaattctctg acgaataatc ttttcttttt tcttttgtaa tagtgtcttt 2520

tgtgtccccc tgttttgagg gatagcaatc ccccaatttg agggatgttt tatccctcgt 2580

tttaggggat tttccctcgt tttgagggat gcaccattct gagatgtttt tatttggtcc 2640

aaacatgccg ccttgctgct tgataatatt cattctgacg agttctaact tggcttcatt 2700

gcaccgtttg acaggtaact ttgtaatctc gctaagttga gaatcggtga ttctgtccat 2760

tggtttattc cacccatagg ttttacgcag aatggcaagc agcactttaa actgtcgctt 2820

ggtcagatct gcgcccgaat aagcctcaag cagcatattt gatagtctgg cgtaaccatc 2880

atcgagatct gccacattac gctcctgtcc ggcaaagtta cctctgccga agttgagtat 2940

ttttgctgta tttgtcataa tgactcctgt tgatagatcc agtaatgacc tcagaactcc 3000

atctggattt gttcagaacg ctcggttgcc gccgggcgtt ttttattggt gagaatcgca 3060

gcaacttgtc gcgccaatcg agccatgtcg tcgtcaacga ccccccattc aagaacagca 3120

agcagcattg agaactttgg aatccagtcc ctcttccacc tgctgatctg cgacttatca 3180

acgcccacag cttccgctgt cttctcagtt ccaagcattg cgattttgtt aagcaacgca 3240

ctctcgattc gtagagcctc gttgcgtttg tttgcacgaa ccatatgtaa gtatttcctt 3300

agataacaat tgattgaatg tatgcaaata aatgcataca ccataggtgt ggtttaattt 3360

gatgcccttt ttcagggctg gaatgtgtaa gagcggggtt atttatgctg ttgttttttt 3420

gttactcggg aagggcttta cctcttccgc ataaacgctt ccatcagcgt ttatagttaa 3480

aaaaatcttt cggcctgcat gaatggcctt gttgatcgcg ctttgatata cgccgagatc 3540

tttagctgtc ttggtttgcc caaagcgcat tgcataatct ttcagggtta tgcgttgttc 3600

catacaacct ccttagtaca tgcaaccatt atcaccgcca gaggtaaaat agtcaacacg 3660

cacggtgtta gatatttatc ccttgcggtg atagatttaa cgtatgagca caaaaaagaa 3720

accattaaca caagagcagc ttgaggacgc acgtcgcctt aaagcaattt atgaaaaaaa 3780

gaaaaatgaa cttggcttat cccaggaatc tgtcgcagac aagatgggga tggggcagtc 3840

aggcgttggt gctttattta atggcatcaa tgcattaaat gcttataacg ccgcattgct 3900

tacaaaaatt ctcaaagtta gcgttgaaga atttagccct tcaatcgcca gagaaatcta 3960

cgagatgtat gaagcggtta gtatgcagcc gtcacttaga agtgagtatg agtaccctgt 4020

tttttctcat gttcaggcag ggatgttctc acctgagctt agaaccttta ccaaaggtga 4080

tgcggagaga tgggtaagca caaccaaaaa agccagtgat tctgcattct ggcttgaggt 4140

tgaaggtaat tccatgaccg caccaacagg ctccaagcca agctagcttc acgctgccgc 4200

aagcactcag ggcgcaaggg ctgctaaagg aagcggaaca cgtagaaagc cagtccgcag 4260

aaacggtgct gaccccggat gaatgtcagc tactgggcta tctggacaag ggaaaacgca 4320

agcgcaaaga gaaagcaggt agcttgcagt gggcttacat ggcgatagct agactgggcg 4380

gttttatgga cagcaagcga accggaattg ccagctgggg cgccctctgg taaggttggg 4440

aagccctgca aagtaaactg gatggctttc ttgccgccaa ggatctgatg gcgcagggga 4500

tcaagatctg atcaagagac aggatgagga tcgtttcgca tgattgaaca agatggattg 4560

cacgcaggtt ctccggccgc ttgggtggag aggctattcg gctatgactg ggcacaacag 4620

acaatcggct gctctgatgc cgccgtgttc cggctgtcag cgcaggggcg cccggttctt 4680

tttgtcaaga ccgacctgtc cggtgccctg aatgaactgc aggacgaggc agcgcggcta 4740

tcgtggctgg ccacgacggg cgttccttgc gcagctgtgc tcgacgttgt cactgaagcg 4800

ggaagggact ggctgctatt gggcgaagtg ccggggcagg atctcctgtc atctcacctt 4860

gctcctgccg agaaagtatc catcatggct gatgcaatgc ggcggctgca tacgcttgat 4920

ccggctacct gcccattcga ccaccaagcg aaacatcgca tcgagcgagc acgtactcgg 4980

atggaagccg gtcttgtcga tcaggatgat ctggacgaag agcatcaggg gctcgcgcca 5040

gccgaactgt tcgccaggct caaggcgcgc atgcccgacg gcgaggatct cgtcgtgacc 5100

catggcgatg cctgcttgcc gaatatcatg gtggaaaatg gccgcttttc tggattcatc 5160

gactgtggcc ggctgggtgt ggcgaccgct atcaggacat agcgttggct acccgtgata 5220

ttgctgaaga gcttggcggc gaatgggctg accgcttcct cgtgctttac ggtatcgccg 5280

ctcccgattc gcagcgcatc gccttctatc gccttcttga cgagttcttc tgagcgggac 5340

tctggggttc gaaatgaccg accaagcgac gcccaacctg ccatcacgag atttcgattc 5400

caccgccgcc ttctatgaaa ggttgggctt cggaatcgtt ttccgggacg ccggctggat 5460

gatcctccag cgcggggatc tcatgctgga gttcttcgcc caccccgggc tcgatcccct 5520

cgcgagttgg ttcagctgct gcctgaggct ggacgacctc gcggagttct accggcagtg 5580

caaatccgtc ggcatccagg aaaccagcag cggctatccg cgcatccatg cccccgaact 5640

gcaggagtgg ggaggcacga tggccgcttt ggtcggatca attcgcgcga ccg 5693

<210> 34
<211> 5693
<212> DNA
<213> Artificial

<220>
<223> artificial sequence

<400> 34
atcgatccgg gcaacgttgt tgccattgct gcaggggggg gggggggggg gggggggggt 60

tgacttccat tgttcattcc acggacaaaa acagagaaag gaaacgacag aggccaaaaa 120

gcctcgcttt cagcacctgt cgtttccttt cttttcagag ggtattttaa ataaaaacat 180

taagttatga cgaagaagaa cggaaacgcc ttaaaccgga aaattttcat aaatagcgaa 240

aacccgcgag gtcgccgccc gtaacctgtc ggatcaccgg aaaggacccg taaagtgata 300

atgattatca tctacatatc acaacgtgcg tggaggccat caaaccacgt caaataatca 360

attatgacgc aggtatcgta ttaattgatc tgcatcaact taacgtaaaa acaacttcag 420

acaatacaaa tcagcgacac tgaatacggg gcaacctcat gtcaaccccc cccccccccc 480

cccccccccc ctgcaggcgg agaactggta ggtatggaag atctctagag aattagcccg 540

cctaatgagc gggctttttt tgaattcgag ctcgcccggg gatcgatcct ctagagtcct 600

gatgcggtat tttctcctta cgcatctgtg cggtatttca caccgcatat ggtgcactct 660

cagtacaatc tgctctgatg ccgcatagtt aagccagtat atacactccg ctatcgctac 720

gtgactgggt catggctgcg ccccgacacc cgccaacacc cgctgacgcg ccctgacggg 780

cttgtctgct cccggcatcc gcttacagac aagctgtgac cgtctccggg agctgcatgt 840

gtcagaggtt ttcaccgtca tcaccgaaac gcgcgaggcc cagctggctt atcgaaatta 900

atacgactca ctataggtcg accggaagct taggatccta tgtattctat agtgtcacct 960

aaatcgtatg tgtatgatac ataaggttat gtattaattg tagccgcgtt ctaacgacaa 1020

tatgtacaag cctaattgtg tagcatctgg cttactgaag cagaccctat catctctctc 1080

gtaaactgcc gtcagagtcg gtttggttgg acgaaccttc tgagtttctg gtaacgccgt 1140

cccgcacccg gaaatggtca gcgaaccaat cagcagggtc atcgctagaa atcatcctta 1200

gcgaaagcta aggatttttt ttatctgaat tctagccaga tccccgggcg agctgcggcc 1260

tgatttatgc tggttactgt tgcgcctgtt agcgcggcaa cgtccggcgc acagaagcta 1320

ttatgcgtcc ccaggtaatg aataattgcc tctttgcccg tcatacactt gctcctttca 1380

gtccgaactt agctttgatt tctgcgatct tcgccagagc ctgtgcacga tttagaggtc 1440

taccgcccat gacaggaagt tgttttactg gttcagggat cgcctcacca cggttaattc 1500

tcgcagtcat atggacaagc tcatctgcgg ccttacggcg taattccgca tcagtaagcg 1560

cattggcccg catgttctga tacaggttgg taaccagcca gtagtgcgcg tttgatttcc 1620

acggataaga ctccgcatcc ggatacaggc ctcgcttccg gcaatactcg taaaccatat 1680

caaccagctc gctgacgttt ggcagtccgg cggtaacgga tgcttcttcc cggcaccatg 1740

caacaaactg cccgggtgat ggcagaaatg gtcgattctg ccgacgggct acgcgcattc 1800

ctgcgttaac ctgttccatc gtggtgatcc cgttttcccg aaaagccaga acccactggc 1860

gacggatttc gttcacttcg ttctggtcac ggttagccag gctcgccggg aaagttgcca 1920

gtaactggct gaacacaccg ttgatgatct gcgctacctg ctgtacctgc ggcttttcgt 1980

cgtactgttc cggcatgttg ttggcgatcc gacgcatctg ctcacggtca aagttaacca 2040

tctgtgcggc gatgtttttc atagatccac cccgtaaatc cagtctgtgt ttgtcaggtc 2100

gagttttggt ttgctggctg tcacgcctgc ctgttgcttg ttacggttga tttcgagttg 2160

ggtccactta tcgcggagtt tggccgggct cagcacgtta ccggaccaga agttgtcctg 2220

gcatgcccag cggaacagca cacacatgtc gcggtggtta cgtccgtcac gttcacgcat 2280

caggcggata tcgttagccc acccagcaaa attcggtttt ctggctgatg gtgcgatagt 2340

cttcaccatg tcaaacatcc actctgcggc ggtcaggtct tctgctgtcc cccacttgct 2400

gccgctctga attgcagcat ccggtttcac cacagaaagg tcgttttctg gctggtcaga 2460

ggattcgcca gaattctctg acgaataatc ttttcttttt tcttttgtaa tagtgtcttt 2520

tgtgtccccc tgttttgagg gatagcaatc ccccaatttg agggatgttt tatccctcgt 2580

tttaggggat tttccctcgt tttgagggat gcaccattct gagatgtttt tatttggtcc 2640

aaacatgccg ccttgctgct tgataatatt cattctgacg agttctaact tggcttcatt 2700

gcaccgtttg acaggtaact ttgtaatctc gctaagttga gaatcggtga ttctgtccat 2760

tggtttattc cacccatagg ttttacgcag aatggcaagc agcactttaa actgtcgctt 2820

ggtcagatct gcgcccgaat aagcctcaag cagcatattt gatagtctgg cgtaaccatc 2880

atcgagatct gccacattac gctcctgtcc ggcaaagtta cctctgccga agttgagtat 2940

ttttgctgta tttgtcataa tgactcctgt tgatagatcc agtaatgacc tcagaactcc 3000

atctggattt gttcagaacg ctcggttgcc gccgggcgtt ttttattggt gagaatcgca 3060

gcaacttgtc gcgccaatcg agccatgtcg tcgtcaacga ccccccattc aagaacagca 3120

agcagcattg agaactttgg aatccagtcc ctcttccacc tgctgatctg cgacttatca 3180

acgcccacag cttccgctgt cttctcagtt ccaagcattg cgattttgtt aagcaacgca 3240

ctctcgattc gtagagcctc gttgcgtttg tttgcacgaa ccatatgtaa gtatttcctt 3300

agataacaat tgattgaatg tatgcaaata aatgcataca ccataggtgt ggtttaattt 3360
gatgcccttt ttcagggctg gaatgtgtaa gagcggggtt atttatgctg ttgttttttt 3420
gttactcggg aagggcttta cctcttccgc ataaacgctt ccatcagcgt ttatagttaa 3480
aaaaatcttt cggcctgcat gaatggcctt gttgatcgcg ctttgatata cgccgagatc 3540
tttagctgtc ttggtttgcc caaagcgcat tgcataatct ttcagggtta tgcgttgttc 3600
catacaacct ccttagtaca tgcaaccatt atcaccgcca gaggtaaaat agtcaacacg 3660
cacggtgtta gatatttatc ccttgcggtg atagatttaa cgtatgagca caaaaaagaa 3720
accattaaca caagagcagc ttgaggacgc acgtcgcctt aaagcaattt atgaaaaaaa 3780
gaaaaatgaa cttggcttat cccaggaatc tgtcgcagac aagatgggga tggggcagtc 3840
aggcgttggt gctttattta atggcatcaa tgcattaaat gcttataacg ccgcattgct 3900
tacaaaaatt ctcaaagtta gcgttgaaga atttagccct tcaatcgcca gagaaatcta 3960
cgagatgtat gaagcggtta gtatgcagcc gtcacttaga agtgagtatg agtaccctgt 4020
tttttctcat gttcaggcag ggatgttctc acctgagctt agaaccttta ccaaaggtga 4080
tgcggagaga tgggtaagca caaccaaaaa agccagtgat tctgcattct ggcttgaggt 4140
tgaaggtaat tccatgaccg caccaacagg ctccaagcca agctagcttc acgctgccgc 4200
aagcactcag ggcgcaaggg ctgctaaagg aagcggaaca cgtagaaagc cagtccgcag 4260
aaacggtgct gaccccggat gaatgtcagc tactgggcta tctggacaag ggaaaacgca 4320
agcgcaaaga gaaagcaggt agcttgcagt gggcttacat ggcgatagct agactgggcg 4380
gttttatgga cagcaagcga accggaattg ccagctgggg cgccctctgg taaggttggg 4440
aagccctgca aagtaaactg gatggctttc ttgccgccaa ggatctgatg gcgcagggga 4500
tcaagatctg atcaagagac aggatgagga tcgtttcgca tgattgaaca agatggattg 4560
cacgcaggtt ctccggccgc ttgggtggag aggctattcg gctatgactg ggcacaacag 4620
acaatcggct gctctgatgc cgccgtgttc cggctgtcag cgcaggggcg cccggttctt 4680
tttgtcaaga ccgacctgtc cggtgccctg aatgaactgc aggacgaggc agcgcggcta 4740
tcgtggctgg ccacgacggg cgttccttgc gcagctgtgc tcgacgttgt cactgaagcg 4800
ggaagggact ggctgctatt gggcgaagtg ccggggcagg atctcctgtc atctcacctt 4860
gctcctgccg agaaagtatc catcatggct gatgcaatgc ggcggctgca tacgcttgat 4920
ccggctacct gcccattcga ccaccaagcg aaacatcgca tcgagcgagc acgtactcgg 4980
atggaagccg gtcttgtcga tcaggatgat ctggacgaag agcatcaggg gctcgcgcca 5040
gccgaactgt tcgccaggct caaggcgcgc atgcccgacg gcgaggatct cgtcgtgacc 5100
catggcgatg cctgcttgcc gaatatcatg gtggaaaatg gccgcttttc tggattcatc 5160
gactgtggcc ggctgggtgt ggcgaccgct atcaggacat agcgttggct acccgtgata 5220

ttgctgaaga gcttggcggc gaatgggctg accgcttcct cgtgctttac ggtatcgccg 5280

ctcccgattc gcagcgcatc gccttctatc gccttcttga cgagttcttc tgagcgggac 5340

tctggggttc gaaatgaccg accaagcgac gcccaacctg ccatcacgag atttcgattc 5400

caccgccgcc ttctatgaaa ggttgggctt cggaatcgtt ttccgggacg ccggctggat 5460

gatcctccag cgcggggatc tcatgctgga gttcttcgcc caccccgggc tcgatcccct 5520

cgcgagttgg ttcagctgct gcctgaggct ggacgacctc gcggagttct accggcagtg 5580

caaatccgtc ggcatccagg aaaccagcag cggctatccg cgcatccatg cccccgaact 5640

gcaggagtgg ggaggcacga tggccgcttt ggtcggatca attcgcgcga ccg 5693

<210> 35
<211> 2030
<212> DNA
<213> Artificial

<220>
<223> artificial sequence

<400> 35
atgcatgaag ctactttgaa gtcgttggaa aacgcgcact tgatggacat tgctgccatc 60

ccacagccaa agaacaaccg caagagacca ggagagccac acatcaagtc tcttgacgag 120

tacaagaagc tgtaccagca gtcgattgag gacccggtga agttctttgg cgaccttgcg 180

aaggagaact tggactggat taaaccattc gacaaggcca agcacccgga tgtcaacagg 240

gggttcctca acaatgacgt tgcgtcgtgg tttattggtg gccagttgaa cgcgtcctac 300

aactgtgttg atagacacgc tttgaagacg ccagacaagc ctgctcttat ctacgaggcc 360

gacgagccag gcgagggacg tatcgtgaca tacgcagagt tgttgaagga ggtttccaaa 420

gttgccgcca tcttgcacga tgccggtgtc cgcaagggcg acaccgttgc agtgtacttg 480

ccaatgattc cagaagccgt tgttacgctc ttggccattg tccgtctcgg tgcattgcac 540

agtgttgtct ttgcagggtt ctcttctgct tctcttagag atcgtatcaa tgatgcaaac 600

tccaagtttg tcgtgaccgc tgatgagtcc aagcgtggtt ccaagaccat tgagacaaag 660

aagattgttg acgaggcgct aaagtcctgt ccacaggtta agaatgtgtt ggtgttcaag 720

cgtactggta accatgttcc attcgactcc aagagagatt tgtggtggca tgaggagacc 780

aagaagtatg gtccatactt cccacctgtc ccagtcaact ctgaggaccc gttgttcctc 840

ctttacacat ctggttccac tggttcacca aagggtatcc agcactgcac cgctgggtac 900

ttgttgagtg ccttgatcac catcaagtac aactttgact tgcataagga ggacattttc 960

ttcactgctg gtgacattgg ttggattacc ggacacacat atgttgttta cggtccattg 1020

ctcaacggtg ccacttctgt tgtctttgaa ggtaccccag cttacccatc ctactctcgt 1080

tactgggaga ttgttgagaa gtacagagtc actcaattct acgttgcccc aactgcattg 1140

agattgttga agagagctgg tgattcctac attgaaggtc atgacttgag ctccttgaga 1200

actcttggtt ctgttggtga accaattgca gccgaggttt gggaatggta cttcgaaaag 1260

attggtaaga aggagatccc aatcttggat acttggtgga tgactgagtc tggttctcaa 1320

ttgatcagtg gtatctctgg tgtcacacca atgaagccag gttctgcctc cttgccattc 1380

tttggtgttg agccttgtat cttggatgca accactggtg aagagctcaa gggtccaaac 1440

gttgaaggta tcttggccat taggtctcca tggccatcca ttgccagaac gatctggaag 1500

aactatgaca gatacttgga cacttatctc aagccatacc caggttactt cttctctggt 1560

gatggtgctg cccgtgatgc tgatggcttc tactggatct tgggtcgtgt tgacgatgtt 1620

gttaacgtgt ctggtcacag attgtccact gctgaaatcg aggctgctct cttggagaac 1680

gatttggttg cagagtctgc tgtcgttggt ttcgcagatg agttgactgg ccaagctgtt 1740

gctgcattcg ttgccctcaa ggacaagaag actgccagtg aacaaggcaa cgcaatctca 1800

aaggacttga tcttgactgt gcgtaaggag attggcccat ttgccgctcc aaagcttatc 1860

atattggttg acgacttgcc aaagaccaga tccggtaaga ttatgagaag aatcttgaga 1920

aagatccttg ctggtgagga ggaccaattg ggtgatgtgt ctacattgtc taacccacag 1980

gttgttgaac agttgatcag tgctgtcaag agcaagaaat gagcggccgc 2030

<210> 36
<211> 2060
<212> DNA
<213> Artificial

<220>
<223> artificial sequence

<400> 36
atgcataatc acaaagttgt gcacgaggcc gctaacgtga aggcgcacca cgctccctca 60

gagttcttcg agaaacagcc aaacaagggc ttcatcaacg acttggagca ctacaaggaa 120

ctgcacaaga agtccatcga ggacccggaa gggttctttg gcccgctggc cagggaatac 180

ctcgactggc acagaccgtt cgacaaggtt gagtccggct ctctggagaa cggtgacgtt 240

gcctggttcc tgaacggtga gctgaacgcg tcctacaact gtgtggatag acacgcgttt 300

gccaacccag gtaagaccgc tatcatctac gaggcggacg atgaaaagga caacagaatc 360

atcacgtttg gtgagctgct gagacaggtg agcgaagttg ccgcggtgct caaatcgtgg 420

ggagttaaga aaggtgacac cgttgctgtc tatttgccta tgattccaga ggccgtagtt 480

gccatgcttg ccgttgccag actcggtgcc atccactctg ttgtgtttgc cggtttctct 540

gctggttctt tgaaagatcg tgtcgttgac gctgattctc acttcatcat cacgtgtgac 600

gaaggtaaga gaggtggtaa gaccgtccac acaaagaaga ttgtcgatga ggccttgaaa 660

gataccgatg tcgtgaagaa gatcctcgtg ttccaaagaa caggctcaga aggcattcca 720

atgaaagctg gccgtgatta ctggtggcat ttggagacca agaagagcag aggctacttg 780

cctccaaccc cagtcaactc tgaggaccca ctcttcctgt tgtacacatc tggttccact 840

ggttcaccaa agggtgtcgt ccacacaacg gctggttact tgctcggtgc cgctttaacc 900

acaagatatg tctttgacat ccatccagag gatattctct tcactgctgg tgatgtcggt 960

tggattaccg gccatacgta tgctctctat ggtccattga ctctcggtac cgctacaatc 1020

atctttgaat ctaccccagc ttatccagat tatggtagat actggagaat cattgaacgt 1080

cataaggcta cccacttcta tgttgcacca actgcattga gattgatcaa gagagttggt 1140

gaggatgaga ttgacaagta tgacatttca tctctgagaa ctttgggttc tgttggagaa 1200

ccaattgccc cagacctttg ggagtggtac cacgagaaga ttggtaagaa caaagccgtg 1260

atctgtgata cgatgtggca aaccgaatcc ggttcccatt tgattgctcc tttggctggt 1320

gcagttccaa ctaagccagg ttcagcaact gttccattct ttggtattga tgcctgtatt 1380

attgatccag ttagtggtaa ggagctagag ggcaatgacg tggaaggtgt ccttgccgtt 1440

aagaagccat ggccatcaat ggctaggtct gtttggaaca accatgacag atacttggag 1500

acttacctca agccataccc aggctactat ttcactggtg atggtgctgg aagagaccat 1560

gacggttact actggattag aggccgtgtc gatgacgttg tcaacgtctc tggtcataga 1620

ttgtccactg cagagattga gaccgcattg agtgatcact tgggagtttc tgaatctgcc 1680

gttgttggta ttccagatga actcactggt caagctgtca ttgcatttgt ctccttgaaa 1740

gatggttact taattcaagc aaatgacaag atcaccgact ctaacgaaca cattacccca 1800

gataagcttc gtcgtgagtt gatcttgcaa gttagaggtg agattggtcc atttgctggt 1860

ccaaagactg ttatcattgt tgacgatcta ccaaagacca gatccggtaa gatcatgaga 1920

agagtcttga gaaagatcac atctaatgaa gctgaccaat tgggagattt gtctacacta 1980

agtaaccctg aagttgttga tcacattatc aaatccgttg acgagcagta cttcaacttg 2040

attaagaagt gagcggccgc 2060

<210> 37
<211> 1223
<212> DNA
<213> Artificial

<220>
<223> artificial sequence

<400> 37
atgcatgaaa acagcgctta tattgttgca gttgccagaa caccaattgg gtccttccaa 60

ggctcccttt catcccagaa ctacgtcgat cttggttctc acgcggtgaa ggctgctctc 120

gccaaagttc cagagatcaa ggccactgat gtcgaggaga ttgtctttgg taacgttgtt 180

caagccaacg ttggtcaaaa cccagctcgt caagttgcca tcaagtctgg catgagcgtc 240

caaaccgtcg cctctaccgt gaacaaggtt tgtgcttctg gtttgaaggc cactatcata 300

gccgcccaga caatcatctt gggtaacgcc gacattgtcg ttgccggtgg tgctgagtcc 360

atgacaaacg ctccatacta cgtaccagct gctcgttctg gtgctcgtta cggtgattcc 420

gtcttggttg atggtttggc ccgtgatggt ttgaaggacg cttacagcga tgatgccatg 480

ggtgtctgtg ctgaaaaatg tgctcgtgac catggcttct ccagagagga ccaggataac 540

tttgcaatcg actcttacaa aaaggcacaa gctgctcatg ctgctggtaa gttcaaatct 600

gagattgccc ctgtcactat caagggtttc agaggtaagc cagatgttgt tgttgaagtt 660

gatgaggaaa gcaccaagtt caacgaacaa aagttgaaga ccgctagaac cgttttccaa 720

aaggagaacg gtaccgtcac cgctgccaat gcttctccaa ttaacgatgg tggtgcagca 780

cttgttctcg tctctcacaa gaagctcgtc gagttgaatt tgaagccaat tgccagaatc 840

ttgggttggg gtgaagccgc acagaaccca gaggatttca ccacatctcc agccttggct 900

gttccaaagg ctttgaagca tgctggtatc accattgacg atgtcgactt ctttgagttc 960

aacgaggcat tctctgttgt tggtttggcc aaccaaaaga tcctcggttt gccaagtgag 1020

aagctcaacg tttacggtgg tgcagttgct attggacacc cattgggatg ctctggtgcc 1080

agaatcttgg tcactttgac atccgtcttg caacaggaag gtggtaagat tggtgttgcc 1140

gctatctgta acggtggtgg tggtgcttct tccatggttt tggagaagtt gtcttctgag 1200

cacgttatct tgtaagcggc cgc 1223

<210> 38
<211> 665
<212> DNA
<213> Artificial

<220>
<223> artificial sequence

<400> 38
atgcattcca agatcatcag attcgagaac ttgagatcat tcttcaagga cggtatgacc 60

atcatgatcg gtggtttctt gaactgtggt acaccaacaa agttgatcga cttcttggtc 120

aacttgaaca tcaagaactt gaccatcatc tccaacgaca cctgttaccc aaacaccggt 180

atcggtaagt tgatctccaa caaccaggtc aagaagttga tcgcctccta catcggttct 240

aacccagata ccggtaagaa gttgttcaac aacgagttgg aggtcgagtt gtccccacaa 300

ggtacattgg tcgagagaat cagagctggt ggttctggtt tgggtggtgt tttgaccaag 360

accggtttgg gtactttgat cgagaagggt aagaagaaga tttccatcaa cggtacagag 420

tacttgttgg agttgccatt gaccgctgac gttgctttga tcaagggttc tatcgttgac 480

gaggccggta acacattcta caagggtact accaagaact tcaacccata catggctatg 540

gccgccaaga cagttatcgt tgaggctgag aacttggttt cctgtgagaa gttggagaag 600

gaaaaggcta tgaccccagg tgtcttgatc aactacatcg ttaaggaacc agcctaagcg 660

gccgc 665

<210> 39
<211> 674
<212> DNA
<213> Artificial

<220>
<223> artificial sequence

<400> 39
atgcataacg acaagaactt ggccaaggaa atcatcgcca agagagttgc cagagagttg 60

aagaacggtc agttggttaa cttgggtgtc ggtttgccaa ccatggttgc tgattacatc 120

ccaaagaact tcaagatcac cttccagtcc gagaacggta tcgttggtat gggtgcttcc 180

ccaaagatca acgaggctga taaggacgtt gttaacgctg gtggtgacta caccaccgtt 240

ttgccagatg gtacattctt cgactcctct gtctcattct ccttgatcag aggtggtcac 300

gttgacgtta cagttttggg tgccttgcag gttgacgaga agggtaacat cgccaactgg 360

atcgttccag gtaagatgtt gtccggtatg ggtggtgcta tggacttggt taacggtgcc 420

aagaaggtca tcatcgctat gagacacacc aacaagggtc agccaaagat tttgaagaag 480

tgtaccttgc cattgaccgc taagtcccag gctaacttga tcgttaccga gttgggtgtc 540

atcgaggtta tcaacgacgg tttgttgttg accgagatca acaagaacac caccatcgac 600

gagatcagat ccttgacagc tgccgacttg ttgatctcca acgagttgag accaatggcc 660

gtttaagcgg ccgc 674

<210> 40
<211> 749
<212> DNA
<213> Artificial

<220>
<223> artificial sequence

<400> 40
atgcatgagt ccgaggtttc caagcaaatc accacaccat tggctgctcc agctttccca 60

agaggtccat acagattcca caacagagag tacttgaaca tcatctacag aaccgacttg 120

gacgccttga gaaagatcgt tccagagcca ttggagttgg acagagccta cgttagattc 180

gagatgatgg ctatgccaga caccacaggt ttgggttctt acacagagtg tggtcaggcc 240

atcccagtta agtacaacgg tgttaagggt gactacttgc acatgatgta cttggacaac 300

gagccagcta tcgccgttgg tagagaatct tctgcctacc caaagaagtt gggttaccca 360

aagttgttcg tcgactccga taccttggtc ggtacattga agtacggtac attgccagtt 420

gctaccgcta ccatgggtta caagcacgaa ccattggact tgaaggaagc ttacgctcag 480

atcgccagac caaacttcat gttgaagatc atccagggtt acgacggtaa gccaagaatc 540

tgtgagttga tctgtgccga gaacaccgat atcacaatcc acggtgcttg gacaggttct 600

gctagattgc agttgttctc ccacgctttg gctccattgg ctgatttgcc agtcttggag 660

atcgtttctg cctctcacat cttgaccgac ttgacattgg gtacaccaaa ggttgtccac 720

gactacttgt ccgttaagta agcggccgc 749

<210> 41
<211> 1064
<212> DNA
<213> Artificial

<220>
<223> artificial sequence

<400> 41
atgcatggtt tcgccatgtt gggtatcaac aagttgggtt ggatcgagaa ggaaagacca 60

gttgctggtt cctacgacgc tatcgttaga ccattggctg tttctccatg tacctctgac 120

atccacaccg ttttcgaagg tgctttgggt gacagaaaga acatgatctt gggtcacgag 180

gccgttggtg aggttgttga agttggttct gaggtcaagg acttcaagcc aggtgacaga 240

gttatcgttc catgtaccac cccagattgg agatccttgg aagttcaagc tggtttccag 300

caacactcca acggtatgtt ggctggttgg aagttctcca acttcaagga cggtgttttc 360

ggtgagtact tccacgttaa cgacgccgat atgaacttgg ccatcttgcc aaaggacatg 420

ccattggaga acgccgttat gatcaccgat atgatgacca caggtttcca cggtgctgag 480

ttggctgata tccaaatggg ttcctccgtt gtcgtcatcg gtatcggtgc tgttggtttg 540

atgggtatcg ctggtgctaa gttgagaggt gctggtagaa tcatcggtgt tggttccaga 600

ccaatctgtg ttgaggctgc taagttctac ggtgctaccg acatcttgaa ctacaagaac 660

ggtcacatcg tcgaccaggt catgaagttg acaaacggta agggtgtcga cagagtcatc 720

atggctggtg gtggttctga gacattgtct caagccgttt ccatggttaa gccaggtggt 780

atcatctcca acatcaacta ccacggttcc ggtgacgctt tgttgatccc aagagttgaa 840

tggggttgtg gtatggctca caagacaatc aagggtggtt tgtgtccagg tggtagattg 900

agagccgaga tgttgagaga catggtcgtc tacaacagag tcgacttgtc caagttggtc 960

acccacgttt accacggttt cgatcacatc gaagaggcct tgttgttgat gaaggacaag 1020

ccaaaggact tgatcaaggc cgtcgtcatc ttgtgagcgg ccgc 1064